# EUROPEAN PATENT APPLICATION

(11) **EP 4 811 382 A1**
(43) Date of publication of application: **23.09.2026**
(21) Application number: 25164917.4
(22) Date of filing: 20.03.2025
(51) Int. Cl.: G16H 30/40, A61B 5/00, A61C 7/00, G16H 50/20, G16H 50/50

(54) **METHOD AND SYSTEM FOR DISPLAYING A USER INTERFACE ASSOCIATED WITH A DENTITION**

(71) Applicant: 3Shape A/S, 1060 Copenhagen K (DK)
(72) Inventor: ALALOUF, Daniella, 1060 Copenhagen K (DK); LUNEAU, Elise, 1060 Copenhagen K (DK); CINELLI, Ester, 1060 Copenhagen K (DK)
(74) Representative: Zacco Denmark A/S

(57) **Abstract**

The present disclosure provides techniques for displaying a user interface associated with a dentition (110). Method (1000) for displaying the user interface includes receiving (1002) scan data (204A) associated with the dentition of a patient (112), generating (1004) a three-dimensional (3D) model of the dentition based on the scan data, determining (1006) anomaly data associated with the dentition based on the 3D model, generating (1008) first interface data (308A) associated with a first user interface (310A) based on the 3D model and/or the anomaly data, and outputting (1010) the first user interface based on the first interface data. The first user interface comprises user-selectable interface elements. Each of the user-selectable interface elements is selectable to generate an anomaly view (702) of the dentition, such that the anomaly view indicates a corresponding dental anomaly within the 3D model.

## Description

### TECHNICAL FIELD

The disclosure relates to dentistry and more particularly relates to a method and a system for displaying an interface associated with a dentition.

### BACKGROUND OF THE INVENTION

A patient's dental structures may be evaluated by a dental practitioner for diagnostic purposes. The evaluation may be based on the practitioner's experience, training, and judgment skills. An individual dental practitioner, doctor, or other such healthcare provider may possess limited experience and diagnosis skills for specific dental diagnoses, anatomical variations, or pathological anomalies. This may also lead to inaccuracies or omissions in diagnostic conclusions or therapeutic recommendations. To overcome such limitations, scanning devices, such as intraoral scanners, have been developed that facilitate the diagnosis process by capturing clear images of dental structures of patients, and enabling dental practitioners to carefully examine the images and conclude better diagnosis results.

A scanning device, for example, an intraoral scanner, is an electronic device that may be used for capturing digital images of a dentition of a patient. The scanning device may be a handheld device used to directly create digital images of the oral cavity. Such digital images may be used in dental procedures, for example, but are not limited to, restorative procedures, orthodontic treatment planning, and periodontics. The scanning devices have become indispensable tools for modern dental practices, contributing to improved treatment outcomes, patient comfort, and overall efficiency.

The scanning device may include, or be connected to, a computer. The scanning device may include a camera to capture images of the dental structures of a patient. These images of the dentitions may be captured during a scanning session. The scanning device may be coupled to a computer that may execute a software that processes digital data sensed by the camera of the scanning device. The software may create a detailed digital impression of the oral cavity. The scanning session may include scan steps, such as, but not limited to, scanning a lower arch of a jaw of the patient, scanning an upper arch of the jaw of the patient, etc.

Typically, a user may use the dental images, or the results provided by the scanning device to diagnose the anomalies present in the dentition of the patient. This analysis may be done manually by the user based on their skills, training, and experience. Even if the analysis is performed by the computer using software(s) automatically, the user may need to analyse the results of the analysis performed by the software. For example, the user may have to conclude using results of the analysis performed by the computer, where reading the results may not be clear and would still require manual skills and expertise.

However, performing a manual analysis of the results may lead to inaccuracies in the conclusions regarding anomalies present in a patient's dentition, and treatment procedures to be followed. If the analysis is performed by the computer automatically, displaying the results of the analysis in a manner that is easily understandable by the user would help in reducing error(s) and ease out the process of reading the results of the analysis and carrying out further procedures for the treatment of the anomalies present in the dentition. This will improve user experience while using the scanning device and will also reduce time and effort made by the user for analyzing the images of the dentition and may further reduce costs incurred by the patient.

Therefore, there is a need for techniques for displaying images associated with a dentition of a patient, such that readability of these images as well as results of analysis/diagnosis of the dentition are enhanced.

### SUMMARY

A computer-implemented method and a system for displaying an interface associated with a dentition is provided.

It is an objective of the present disclosure to provide techniques for accurate and real-time identification of anomalies present in the dentition of a patient.

It is another objective of the present disclosure to provide an interface for dental image analytics that is easily understandable by a user and that guides the user in a dental diagnostic process.

It is yet another objective of the present disclosure to provide techniques for reducing error(s) and easing out the process of reading the results of dental image analysis to implement procedures for treatment of anomalies in the dentition of the patient.

In one aspect, a computer-implemented method for displaying an interface associated with a dentition is provided. The method may include receiving scan data associated with the dentition of a patient. The method may further include generating a three-dimensional (3D) model of the dentition based on the scan data. The method may further include determining anomaly data associated with the dentition of the patient. The anomaly data may be associated with at least one of a plurality of dental anomalies. The method may further include generating first interface data associated with a first user interface based on at least one of the 3D model and/or the anomaly data. The first user interface may comprise one or more user-selectable interface elements. Each of the one or more user-selectable interface elements is selectable to generate an anomaly view of the dentition, such that the anomaly view indicates a corresponding dental anomaly of the plurality of dental anomalies within the 3D model. The method may further include outputting the first user interface based on the first interface data.

In accordance with some example embodiments, the computer-implemented method may further include receiving a user selection of a first dental anomaly of the plurality of dental anomalies, wherein the user selection is received based on the outputting of the first user interface. The method may further include generating a first anomaly view of the dentition, wherein the first anomaly view comprises at least a first portion of the 3D model, and wherein at least the first portion of the 3D model indicates an occurrence of the first dental anomaly in the dentition of the patient. The method may further include outputting the first anomaly view.

In accordance with some example embodiments, the computer-implemented method may further include causing to display, via a display device, the first anomaly view, such that the first anomaly view is overlaid on the first user interface. In stead of overlaying, the first anomaly view may be replaced by another view.

In accordance with some example embodiments, generating the first anomaly view may comprise visually modifying the at least the first portion of the 3D model within the 3D model.

In accordance with some example embodiments, the anomaly data may indicate an anomaly score associated with at least one dental anomaly of the plurality of dental anomalies. The computer-implemented method may further include receiving first visual representation data associated with the plurality of dental anomalies. The first visual representation data may indicate a plurality of first visual representations. Each of the plurality of first visual representations may be associated with a dental anomaly of the plurality of dental anomalies. The method may further include generating second visual representation data associated with the plurality of dental anomalies based on the anomaly data. The second visual representation data may indicate a plurality of second visual representations of the anomaly score of the corresponding dental anomaly of the plurality of dental anomalies. The method may further include generating the first interface data based on the first visual representation data and the second visual representation data.

In accordance with some example embodiments, generating the second visual representation data associated with a specific second visual representation of the plurality of second visual representations may include determining an anomaly score associated with a specific dental anomaly of the plurality of dental anomalies based on the anomaly data associated with the specific dental anomaly. The generating of the second visual representation data may further include generating categorization data associated with the specific second visual representation for the specific dental anomaly based on the anomaly score. The categorization data may indicate at least one of: one or more division areas of the specific second visual representation, a severity category from a plurality of severity categories for each of the one or more division areas, or a visual indicator from a plurality of visual indicators for each of the one or more division areas. The generating of the second visual representation data may further include generating the second visual representation data associated with the specific dental anomaly based on the categorisation data.

In accordance with some example embodiments, each interface element of the one or more user-selectable interface elements may indicate at least one of: a first visual representation of the plurality of first visual representations, or a second visual representation of the plurality of second visual representations, based on corresponding dental anomaly of the plurality of dental anomalies.

In accordance with some example embodiments, each first visual representation of the plurality of first visual representations may indicate a symbol associated with the corresponding dental anomaly of the plurality of dental anomalies. Also, each second visual representation of the plurality of second visual representations may indicate a scale for the anomaly score of the corresponding dental anomaly of the plurality of dental anomalies.

In accordance with some example embodiments, the computer-implemented method may further include determining a timestamp associated with the first interface data. The computer-implemented method may further include storing the scan data, the 3D model, the anomaly data, and the first interface data in association with the timestamp.

In accordance with some example embodiments, the computer-implemented method may further include generating report data based on the anomaly data and the first interface data, wherein the report data indicates at least one of: a health status of the dentition, or a treatment for at least one dental anomaly of the plurality of dental anomalies. The computer-implemented method may further include storing the report data in association with the timestamp.

In accordance with some example embodiments, the computer-implemented method may further include generating second interface data associated with a timeline of one or more historical dental examinations of the dentition, wherein the second interface data may comprise one or more user-selectable timestamp interface elements, and wherein each of the one or more user-selectable timestamp interface elements is selectable to output historical interface data associated with one of the one or more historical dental examinations of the dentition. The computer-implemented method may further include outputting the timeline based on the second interface data.

In accordance with some example embodiments, the computer-implemented method may further include causing to display, via a display device, a second user interface associated with the timeline based on the second interface data, wherein the second user interface is displayed with first user interface.

In another aspect, a computer system for displaying an interface associated with a dentition is provided. The computer system may include a memory configured to store computer-executable instructions. The computer system may include one or more processors configured to execute the instructions. The one or more processors, by executing the instructions, may be configured to receive scan data associated with a dentition of a patient. The one or more processors may further configured to generate a three-dimensional (3D) model of the dentition based on the scan data. The one or more processors may further be configured to determine anomaly data associated with the dentition of the patient. The anomaly data may be associated with at least one of a plurality of dental anomalies. The one or more processors may further be configured to generate first interface data associated with a first user interface based on at least one of the 3D model or the anomaly data. The first user interface may comprise one or more user-selectable interface elements. Each of the one or more user-selectable interface elements is selectable to generate an anomaly view of the dentition, such that the anomaly view indicates a corresponding dental anomaly of the plurality of dental anomalies within the 3D model. The one or more processors may further be configured to output the first user interface based on the first interface data.

In accordance with some example embodiments, the computer system may include a display device. The one or more processors are further configured to execute the instructions to receive a user selection of a first dental anomaly of the plurality of dental anomalies. The user selection may be received based on the output of the first user interface. The one or more processors may further be configured to generate a first anomaly view of the dentition. The first anomaly view may comprise at least a first portion of the 3D model. Also, at least the first portion of the 3D model may indicate an occurrence of the first dental anomaly in the dentition of the patient. The one or more processors may be further configured to output the first anomaly view.

In another aspect, a computer program product for displaying an interface associated with a dentition is provided. The computer program product may include one or more computer-readable storage media, and program instructions stored on the one or more computer-readable storage media. The program instructions may perform operations. The operations may include receiving scan data associated with the dentition of a patient. The operations may further include generating a three-dimensional (3D) model of the dentition based on the scan data. The operations may further include determining anomaly data associated with the dentition of the patient. The anomaly data may be associated with at least one of a plurality of dental anomalies. The operations may further include generating first interface data associated with a first user interface based on at least one of the 3D model or the anomaly data. The first user interface may comprise one or more user-selectable interface elements. Each of the one or more user-selectable interface elements is selectable to generate an anomaly view of the dentition, such that the anomaly view indicates a corresponding dental anomaly of the plurality of dental anomalies within the 3D model. The operations may further include outputting the first user interface based on the first interface data.

The foregoing summary is illustrative only and is not intended to be in any way limiting. In addition to the illustrative aspects, embodiments, and features described above, further aspects, embodiments, and features will become apparent by reference to the drawings and the following detailed description.

### EFFECT(S) OF THE INVENTION

According to the present disclosure, a computer-implemented method, a system, and a computer program product for displaying an interface associated with a dentition are provided. In an example, dental image analytics of the dentition of a patient may be obtained using a processor or a computing device associated with a scanning device during a scanning session of the patient. The present disclosure provides an effective way to accurately determine anomalies present in the dentition of the patient. For example, during a scanning session, the dental images of the dentition may be captured. These dental images may correspond to the dentition or the oral cavity of the patient. The dentition may include, but is not limited to, an upper jaw and a lower jaw. Further, the embodiments of the present disclosure provide techniques to accurately display the portions of the dentition. Some portions of the dentition may be affected by one or more anomalies. The portions of the dentition that are affected by, for example, a selected anomaly as per the user's desire, may be displayed separately while hiding other portions of the dentition that may not affected, or may be affected by any anomaly other than the selected anomaly.

The interface element associated with the dentition enables the user to easily understand the anomalies present in the dentition, for example, their location, number of teeth affected, severity, and the like. The interface element may be shown via a display device. The display device may include, but is not limited to, a screen, a monitor, a plasma display, light emitting diode (LED) display, etc. Displaying the results of the analysis of the dentition of the patient within the interface element makes the analysis easy to understand by the user, may help in reducing error(s), and ease out the process of reading the results of the analysis and carrying out further procedures for the treatment of the anomalies present in the dentition. In addition, displaying accurate results in a manner described in the present disclosure would further reduce the time and efforts made by the user to analyse the dentition, and in turn, reduce costs incurred by the patient.

### BRIEF DESCRIPTION OF THE DRAWINGS

The present disclosure is illustrated by way of example and not by way of limitation in the figures of the accompanying drawings, in which the like reference numerals indicate like elements and in which:
FIG. 1 illustrates a network environment in which a computer system for displaying a user interface associated with a dentition is implemented, in accordance with an embodiment of the disclosure;
FIG. 2 illustrates a block diagram of the computer system of FIG. 1, in accordance with an embodiment of the disclosure;
FIG. 3 illustrates a block diagram of exemplary operations for displaying the user interface associated with the dentition, in accordance with an embodiment of the disclosure;
FIG. 4A shows a first exemplary variation of a first user interface associated with the dentition, in accordance with an embodiment of the disclosure;
FIG. 4B shows a second exemplary variation of the first user interface associated with the dentition, in accordance with an embodiment of the disclosure;
FIG. 4C shows a third exemplary variation of the first user interface associated with the dentition, in accordance with an embodiment of the disclosure;
FIG. 4D shows a fourth exemplary variation of the first user interface associated with the dentition, in accordance with an embodiment of the disclosure;
FIG. 4E shows an exemplary variation of legend information associated with the first user interface associated with the dentition, in accordance with an embodiment of the disclosure;
FIG. 5A shows a first exemplary variation of severity overview information associated with the first user interface associated with the dentition, in accordance with an embodiment of the disclosure;
FIG. 5B shows a second exemplary variation of the severity overview information associated with the first user interface, in accordance with an embodiment of the disclosure;
FIG. 5C shows a third exemplary variation of the severity overview information associated with the first user interface, in accordance with an embodiment of the disclosure;
FIG. 6A shows a fifth exemplary variation of the first user interface associated with the dentition, in accordance with an embodiment of the disclosure;
FIG. 6B shows a sixth exemplary variation of the first user interface associated with the dentition, in accordance with an embodiment of the disclosure;
FIG. 7A shows a first exemplary variation of an anomaly view associated with the first user interface, in accordance with an embodiment of the disclosure;
FIG. 7B shows a second exemplary variation of the anomaly view associated with the first user interface, in accordance with an embodiment of the disclosure;
FIG. 7C shows a third exemplary variation of the anomaly view associated with the first user interface, in accordance with an embodiment of the disclosure;
FIG. 8A is a flowchart that illustrates exemplary operations for outputting the second user interface, in accordance with an embodiment of the disclosure;
FIG. 8B shows an exemplary variation of a second user interface associated with a timeline, in accordance with an embodiment of the disclosure;
FIGs. 9A-9D show various exemplary elements of report data that may be included in a report associated with the second user interface, in accordance with various embodiments of the disclosure;
FIG. 10 is a flowchart that illustrates an exemplary method for displaying the first user interface associated with the dentition, in accordance with an embodiment of the disclosure;
FIG. 11 is a flowchart that illustrates exemplary operations for outputting the anomaly view, in accordance with an embodiment of the disclosure;
FIG. 12 is a diagram depicting an exemplary method for generating the report, in accordance with an embodiment of the disclosure;
FIG. 13 is a diagram depicting an overview of scanning of the dentition, in accordance with an embodiment of the disclosure;
FIG. 14 is a diagram depicting an exemplary 3D model of a portion of the dentition, in accordance with an embodiment of the disclosure;
FIG. 15A is a diagram that illustrates a first set of exemplary operations of a trained machine learning model for determining a dental anomaly, in accordance with an embodiment of the disclosure; and
FIG. 15B is a diagram that illustrates a second set of exemplary operations of a trained machine learning model for determining the dental anomaly, in accordance with an embodiment of the disclosure.

### DETAILED DESCRIPTION

In the following description, for purposes of explanation, numerous specific details are set forth in order to provide a thorough understanding of the present disclosure. It will be apparent, however, to one skilled in the art that the present disclosure may be practiced without these specific details. In other instances, systems and methods are shown in block diagram form only to avoid obscuring the present disclosure.

Some embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. Also, reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification does not necessarily all refer to the same embodiment, nor are separate or alternative embodiments mutually exclusive of other embodiments. Further, the terms "a" and "an" herein do not denote a limitation of quantity but rather denote the presence of at least one of the referenced items. Moreover, various features are described which may be exhibited by some embodiments and not by others. Similarly, various requirements are described which may be requirements for some embodiments but not for other embodiments.

The embodiments are described herein for illustrative purposes and are subject to many variations. It is understood that various omissions and substitutions of equivalents are contemplated as circumstances may suggest or render expedient but are intended to cover the application or implementation without departing from the spirit or the scope of the present disclosure. Further, it is to be understood that the phraseology and terminology employed herein are for the description and should not be regarded as limiting. Any heading utilized within this description is for convenience only and has no legal or limiting effect.

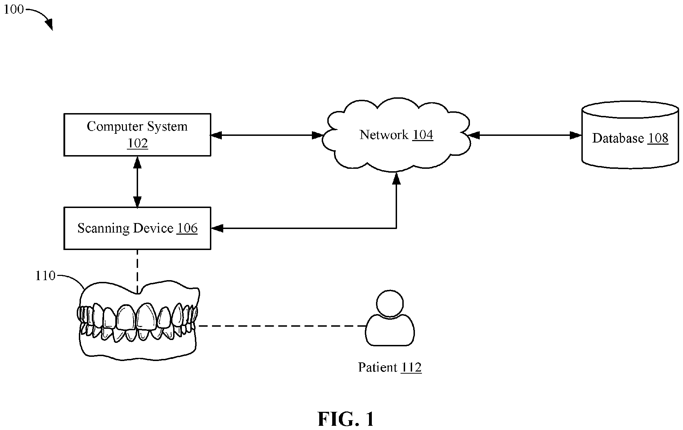

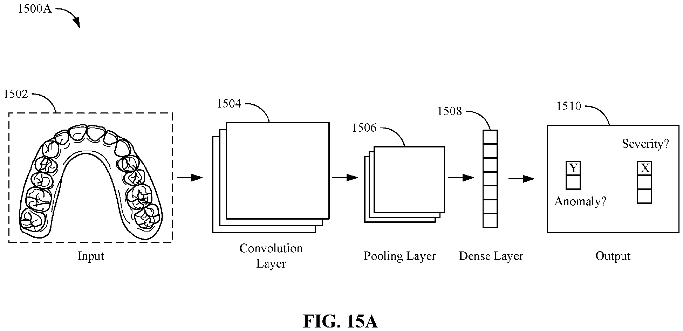

As used in this specification and claims, the terms "for example" "for instance" and "such as", and the verbs "comprising," "having," "including" and their other verb forms, when used in conjunction with a listing of one or more components or other items, are each to be construed as open-ended, meaning that that the listing is not to be considered as excluding other, additional components or items. Other terms are to be construed using their broadest reasonable meaning unless they are used in a context that requires a different interpretation. Turning now to FIG. 1 - FIG. 15B, a brief description concerning the various components of the present disclosure will now be briefly discussed. Reference will be made to the figures showing various embodiments of displaying an interface associated with a dentition.

A computer-implemented method and a system for displaying an interface associated with a dentition (also referred to as an oral cavity) is provided. The interface may be generated using scan data. The scan data may include images of the dentition. For obtaining the scan data of the dentition of a patient, a dental scanning device such as, but not limited to, an intraoral scanner may be used. The scan data including images of the dentition (also referred to as dental images) may be processed to generate a three-dimensional (3D) model of the dentition of the patient. The 3D model may be analysed to determine anomaly data indicating one or more dental anomalies in the dentition. The one or more dental anomalies may include pathologies, such as surface caries, tooth wear, gingival recession, plaque formation, proximal caries, gingivitis, etc. Further, interface data associated with a user interface may be generated based on the 3D model and/or the anomaly data. The user interface may include one or more user-selectable interface elements. To this end, each of the one or more user-selectable interface elements may be selected to generate an anomaly view of the dentition. The anomaly view may indicate a corresponding dental anomaly within the 3D model. The user interface may be output based on the interface data.

FIG. 1 illustrates a network environment 100 in which a computer system 102 (also referred to as a system 102) for displaying a first user interface associated with a dentition 110 of a patient 112, in accordance with an embodiment of the disclosure is shown. The network environment 100 includes a scanning device 106 for scanning the dentition 110 of the patient 112. The scanning device 106 may be connected with the system 102. The network environment 100 includes a database 108 connected to the system 102 and the scanning device 106 via a network 104.

The system 102 may include suitable logic, circuitry, interfaces, and/or code that may be configured to display the first user interface associated with the dentition 110 of the patient 112. In an embodiment, the system 102 is configured to receive scan data of a dental scan of the dentition 110 from the scanning device 106. Examples of the scanning device 106 may include, but are not limited to, an intraoral scanner, a cone beam computed tomography (CBCT) device, a digital panoramic X-ray machine, an optical scanner, a laser scanner, and a mobile dental scanner.

In operation, the system 102 is configured to receive scan data that may be generated by the scanning device 106. Particularly, for the generation of the scan data, a user, such as a dental practitioner, of the scanning device 106 may initiate a scanning session of the dentition 110 of the patient 112 by positioning the scanning device 106 aligned with the dentition 110. In an exemplary embodiment, the scanning session may be performed in a dental clinic. In another exemplary embodiment, the scanning process may be performed at a home of the patient 112.

In an example, the scanning device 106 may include or be connected to a web server that may be configured to communicate via a web network and establish a connection with the network 104. In an example, the scanning device 106 may be configured to provide the 3D surface information based on the visible light information and/or the images of the dentition 110 to the system 102. The scanning device 106 may further include a processing unit, a memory unit, a communication interface, and additional components. The processing unit, the memory unit, the communication interface, and the additional components of the scanning device 106 may be communicatively coupled to each other. In an example, the processing unit of the scanning device 106 may be configured to detect NIR and visible light during the scanning session of teeth of the patient 112. The detected NIR and visible light may be used to generate a plurality of two-dimensional (2D) images, for example, 2D NIR images, and 2D white light images. The plurality of 2D images may include images of the dentition 110 including teeth of the patient 112 from various angles or viewing points. The scanning device 106 may include various modalities to record information. Examples of the various modalities include, but are not limited to, colour scanning mode to acquire colour information of teeth and gingiva, infrared scanning mode to acquire infrared data representing sub-surface information of the teeth, UV scanning mode to record fluorescence emitted from bacteria present on teeth and gingiva.

In an example, in-focus measurements in the white light images may be determined, and projected features of the teeth in the dentition 110 may be tracked across the white light images. For example, a computation function may be performed or solved to triangulate depth information for the teeth and determine the 3D surface information for the teeth in the dentition 110. Further, the system 102 may generate the 3D model of the dentition 110 based on the determined 3D surface information of the dentition 110.

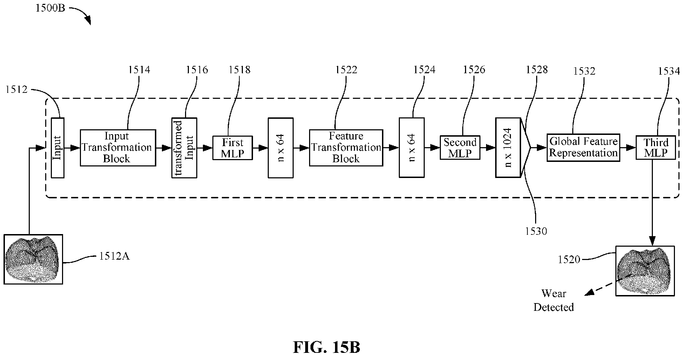

Further, the system 102 is configured to generate a 3D model of the dentition 110 based on the scan data. In an embodiment, the system 102 may generate the 3D model of the dentition 110 using one or more machine learning (ML) models. In this regard, the system 102 may input the scan data to the ML models responsible for generating the 3D model of the dentition 110 based on the scan data associated with the patient 112. The ML models for generating the 3D model of the dentition 110 may be trained based on historical scan data of various patients during various scanning sessions. An exemplary 3D model of an exemplary dentition is shown with reference to FIG. 15. In another embodiment, the system 102 may generate the 3D model of the dentition 110 by "stitching" so-called sub-scans comprised of plurality of 2D images of the scan data.

The scanning device 106 may be a device that may be used in dentistry. The scanning device 106 may provide a digital alternative to the traditional method of taking an analog impression of a dental object, such as the dentition 110. The scanning device 106 may capture 3D digital dental impressions and use the data to produce a highly detailed image of dentition 110 of the patient 112. In an example, the scanning device 106 may project light from a light source onto the dentition 110, such as full dental arches of the patient 112, and other parts of the dentition 110 of the patient 112. The light source may project, for example, visible light, white light wavelength pulses, or near-infrared (NIR) wavelength pulses onto the dentition 110. Further, scan data generated by the scanning device 106 may include images of the dentition 110. The scan data may be in the form of two-dimensional (2D) images. The scanning device 106 is configured to transmit the scan data to the database 108 or the system 102 via the network 104. Further, system 102 may use the scan data to generate the 3D model of the dentition 110.

Based on the scan data such as the 2D images of the dentition 110, the system 102 may be configured to determine the 3D surface information of the teeth of the patient 112. The 3D surface information may be a digital representation of the dental arch of the teeth of the patient 112 depicted in a 3D space. The 3D surface information may include, for example, 3D point cloud data corresponding to white light images. The 3D point cloud data may correspond to 3D real-world coordinates. The 3D surface information may additionally include colour and texture reflected from the surface of the teeth.

Further, the system 102 is configured to determine anomaly data associated with the dentition 110 of the patient 112. For determining the anomaly data, the system 102 may be configured to analyse the scan data by generating the three-dimensional (3D) model of the dentition 110. Thereafter, the system 102 is configured to apply trained machine learning (ML) model(s) for determining the anomaly data. The ML model(s) may be trained based on training data that may be developed using human inputs, for example, manual inputs provided by one or more users. Further, the system 102 is configured to generate interface data associated with the first user interface based on the 3D model and/or the anomaly data. The interface data may be based on anomaly data. In an example, the system 102 determines anomaly data indicating 7 teeth out of a total of 32 teeth in the dentition 110 suffer from surface caries anomaly. In such a case, the interface data may include icon(s) and/or other graphical representations representing the 7 teeth suffering from surface caries anomaly. In an embodiment, the system 102 may be configured to output the first user interface associated with the dentition 110 of the patient 112.

In an embodiment, the anomaly data may be associated with at least one of a plurality of dental anomalies. The plurality of dental anomalies may include, but are not limited to, a surface caries anomaly, an occlusal caries anomaly, a proximal caries anomaly, a tooth wear anomaly, a gingival recession anomaly, a gingivitis anomaly, and a plaque anomaly. The surface caries anomaly may refer to localized decay of tooth enamel caused by acid produced by bacteria that feed on sugars. The surface caries anomaly may appear as white spots or discolouration on the tooth surface and can progress to more severe decay. The occlusal caries anomaly may refer to dental decay that occurs on the chewing surfaces of the molars and premolars, where food particles and plaque can accumulate in the pits and fissures and may lead to pain and/or sensitivity. The proximal caries anomaly may refer to a dental anomaly that occurs on the surfaces of teeth that are adjacent to one another, for example, between molars and premolars, and may lead to tooth sensitivity, pain, and potential loss of tooth structure. The tooth wear anomaly may refer to the gradual loss of tooth structure due to various factors, including but not limited to, abrasion (from brushing or grinding), erosion (from acidic foods or beverages), and attrition (from tooth-to-tooth contact). The tooth wear anomaly may lead to sensitivity, changes in bite alignment, and aesthetic concerns as the enamel thins and dentin becomes exposed. The gingival recession anomaly may refer to gradual loss of gum tissue, resulting in the exposure of the tooth roots. The gingival recession anomaly may be caused by factors such as, but not limited to, periodontal disease, aggressive brushing, or genetic predisposition. The gingival recession anomaly may lead to tooth sensitivity and a higher risk of decay at the root surface. The gingivitis anomaly may refer to a dental anomaly characterized by inflammation of the gums, typically caused by the accumulation of plaque and bacteria on the teeth. The plaque anomaly may refer to formation of a sticky, colourless biofilm that forms on teeth, composed of bacteria, food particles, and saliva, and may lead to tooth decay, gingivitis, and periodontal disease.

The system 102 may be configured to perform diagnostics on the 3D model of the dentition 110. In an embodiment, after the generation of the 3D model of the dentition 110 of the patient 112, the system 102 is configured to automatically diagnose the dentition 110 to detect one or more dental anomalies that may be present in the dentition 110. For automatically diagnosing the dentition, the system 102 may use one or more trained ML models. In an embodiment, trained ML models may be used to detect a specific anomaly from the plurality of anomalies separately. In another embodiment, a single trained ML model may be used to detect the one or more dental anomalies in the dentition 110. In an embodiment, an input to the trained ML model(s) may be in the form of individual teeth in point cloud data format. The point cloud data format is a data structure used to represent a collection of points in a 3D space, where each point is defined by its coordinates (for example, X, Y, and Z coordinates) and may also include additional attributes such as colour, intensity, or other measurements. In an alternate embodiment, the input to the trained ML model(s) may be in the form of 2D images of the 3D model, and the trained ML model(s) may process the 2D images of the 3D model. ML model(s) may be trained based on a previously labelled training dataset comprising a plurality of 3D models of dentitions labelled with dental anomalies. The training dataset may comprise more than 500 labelled 3D models of dentitions, preferably more than 1000 labelled 3D models of dentitions, and more preferably more than 2000 labelled 3D models of dentitions. Further, the trained ML model(s) may output probabilities that may show whether a dental anomaly among the plurality of dental anomalies is detected in the dentition 110 or not. Further, the output of the ML model(s) may also indicate severity information for each detected dental anomaly in the dentition 110. For example, the severity information may indicate a category such as, but not limited to, initial, moderate, severe, etc. in which the detected anomaly may fall.

During the scanning session of the dentition 110 in real-time or after completion of the scanning session of the patient 112 by the scanning device 106, the system 102 may generate a 3D model of the dentition 110 from the scan data to determine the anomaly data. For example, the system 102 may determine the dental anomaly for each of the scanned portions of the dentition 110. For example, the system 102 may be configured to determine, for every tooth in the dentition 110, various aspects of dental anomalies. Individual teeth in the 3D model of the dentition 110 may be recognized through known segmentation processes where individual objects such as teeth or gingiva are identifed. The various aspects of the dental anomalies for each tooth include, for example, whether a portion of the tooth is affected by one or more dental anomalies or not, if a portion of the tooth is affected by the one or more dental anomalies, then which portion of the tooth is affected by which dental anomaly, and the extent of the dental anomaly with which the portion of the tooth is affected, etc. In an embodiment, the system 102 may utilize the ML models to analyse the 3D model of the dentition 110 to determine the anomaly data of the scanned portion of the dentition 110. Pertinently, the ML models responsible for determining the anomaly data from the 3D model of the dentition 110 are different from the ML model responsible for generating the 3D model of the dentition 110 from the scan data of the patient 112. The ML model(s) may be trained on 3D models of various dentitions associated with a plurality of patients and/or the 3D model of the dentition 110 associated with the patient 112. Based on the processing, the ML model(s) may generate an output including the anomaly data associated with the dentition 110 of the patient 112.

In an embodiment, the system 102 is configured to generate first interface data associated with the first user interface based on at least one of the 3D model or the anomaly data. The first user interface includes one or more user-selectable interface elements. Moreover, each interface element of the one or more user-selectable interface elements is selectable to generate an anomaly view of the dentition 110. For example, the anomaly view indicates a corresponding dental anomaly of the plurality of dental anomalies within the 3D model.

Based on the anomaly data, the system 102 may generate the first interface data. For example, the anomaly data indicates that the dentition 110 has tooth T1 and tooth T2 affected with anomaly DA1, and tooth T3, tooth T4, and tooth T5 affected with anomaly DA2. In such case, the interface data may include a first user-selectable interface element IE1 corresponding to the anomaly DA1 and a second user-selectable interface element IE2 corresponding to the anomaly DA2. The interface elements IE1 and IE2 may appear on the first user interface. Also, when the user selects one of the user-selectable interface elements IE1 or IE2, then the anomaly view of the dentition 110 corresponding to DA1 or DA2, respectively, may be generated and output. For example, a user selects interface element IE1 on the first user interface, then a first anomaly view may be generated corresponding to the anomaly DA1. The first anomaly view may include a highlighted portion of the dentition 110, where the highlighted portion is affected by the anomaly DA1. In case the user selects both the interface elements IE1 and IE2, an anomaly view of the dentition 110 on the first user interface corresponding to both the anomalies DA1 and DA2 may be generated and output by the system 102. Such an anomaly view corresponding to both the anomalies DA1 and DA2 may include a highlighted portion(s) of the dentition 110 where the anomalies DA1 and DA2 may be occurring.

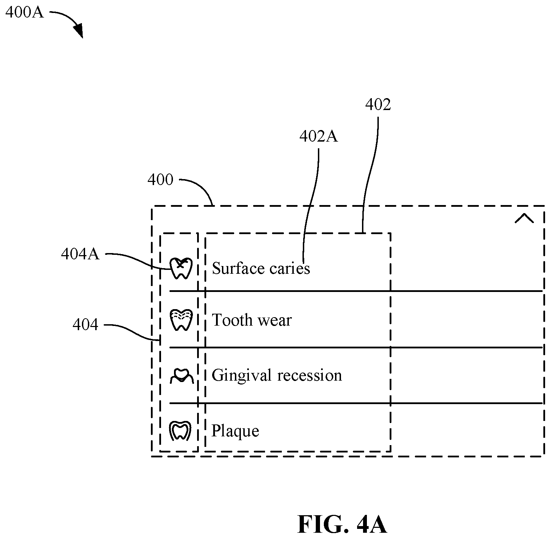

Further, the system 102 may be configured to output the generated first user interface. The output of the generated first user interface may encompass the display of the generated first user interface via a display device associated with the system 102. For example, the first user interface may include the 3D model of the dentition 110 of the patient 112 and the user-selectable interface elements based on the anomaly data. Upon selection by the user of any of the user-selectable interface elements, the first user interface may include the anomaly view of the dentition 110 corresponding to the selected user-selectable interface element. Some exemplary variations of various exemplary components that may be shown at the first user interface are provided in FIGs. 4A-4E, FIGs. 5A-5C, FIGs. 6A-6B, FIGs. 7A-7C, and FIGs. 8A- 8B of the disclosure.

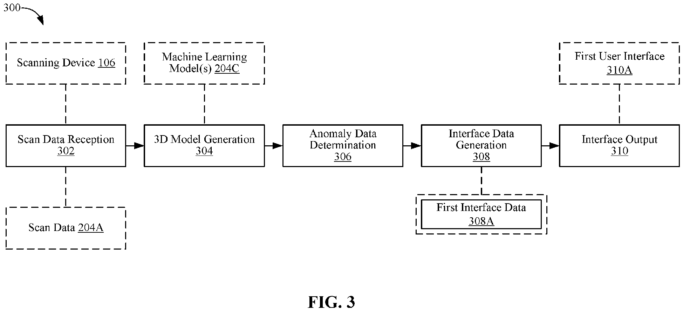

In an example, the system 102 is configured to render the first user interface on the display device to provide anomaly information pertaining to the dentition 110 to a user, such as the patient 112, a clinician, a dentist, and the like. In another example, the system 102 is configured to output the first user interface for downstream processing, such as generation of a record of the patient 112 for record-keeping or storage, identification of treatments for the patient 112, generation of prosthetic or orthodontic parts for the patient 112, etc. Details of the first user interface are provided further with reference to FIG. 3 in this disclosure.

The database 108 may be an organised collection of data that may be received from the system 102 and/or the scanning device 106 over the network 104, and/or data that may be pre-stored in the database 108 for use by the system 102. Examples of the data in the database 108 may include, but not limited to, scan data from the scanning device 106, output of the system 102, reports generated by the system 102, information for accessing software(s) for use by system 102 for processing the scan data, etc. Within the database 108, the data may be organised in the form of one or more tables. The one or more tables may include rows, columns, and indexes to make the data more relevant. In an embodiment, the database 108 may be of a type such as, but not limited to, a cloud database, a centralized database, or an operational database.

The network 104 may be wired, wireless, or any combination of wired and wireless communication networks, such as cellular, Wi-Fi, internet, local area networks, or the like. In some embodiments, the network 104 may include one or more networks such as a data network, a wireless network, a telephony network, or any combination thereof. It is contemplated that the data network may be any local area network (LAN), metropolitan area network (MAN), wide area network (WAN), a public data network (e.g., the Internet), short-range wireless network, or any other suitable packet-switched network, such as a commercially owned, proprietary packet-switched network, e.g., a proprietary cable or fibre-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks (e.g. LTE-Advanced Pro), 5G New Radio networks, ITU-International Mobile Communication (IMT) 2020 networks, code division multiple access (CDMA), wideband code division multiple access (WCDMA), wireless fidelity (Wi-Fi), wireless LAN (WLAN), Bluetooth, Internet Protocol (IP) data casting, satellite, mobile ad-hoc network (MANET), and the like, or any combination thereof.

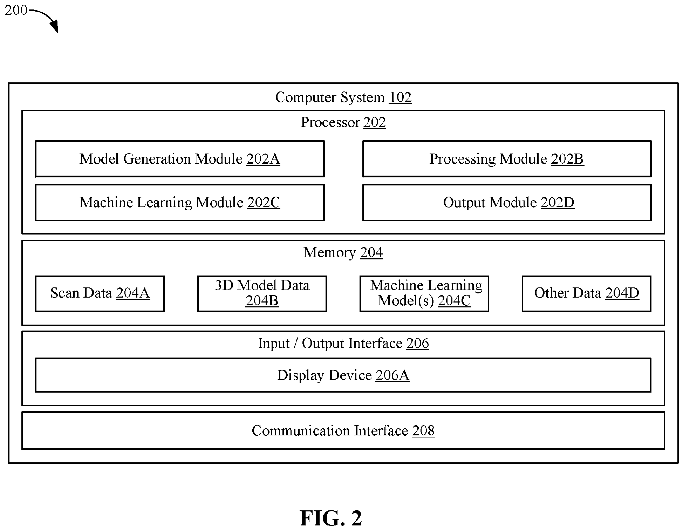

FIG. 2 illustrates a block diagram 200 of the computer system 102 of FIG. 1, in accordance with an embodiment of the disclosure. FIG. 2 is explained in conjunction with FIG. 1. The system 102 may include one or more processors 202 (referred to as a processor 202, hereinafter), one or more non-transitory memory 204 (referred to as a memory 204, hereinafter), an input/output (I/O) interface 206, and a communication interface 208, and the display device 206A. The processor 202 may include modules, depicted as, a model generation module 202A, a processing module 202B, a machine learning module 202C, and an output module 202D. The processor 202 may be connected to the memory 204, the I/O interface 206 and the communication interface 208 through wired or wireless connections. Although in FIG. 2, it is shown that the system 102 includes the processor 202, the memory 204, the I/O interface 206, and the communication interface 208, however, the disclosure may not be so limiting and the system 102 may include fewer or more components to perform the same or other functions of the system 102.

In an embodiment, the model generation module 202A and the output module 202D may be integrated within the I/O interface 206. In some embodiments, the model generation module 202A may receive input data (such as scan data and/or user input data), and the output module 202D may output processed data (such as anomaly view of the dentition 110, the first user interface, etc.), via the I/O interface 206. In an embodiment, the I/O interface 206 may include a display device 206A.

In accordance with an embodiment, the system 102 may store data that may be generated by the modules while performing corresponding operations or may be retrieved from a database, such as the database 108 associated with the system 102. For example, the data may include scan data 204A that may be generated during a scanning session using the scanning device 106, 3D model data 204B that may be generated by the system 102 using the scan data 204A, etc.

The processor 202 of the system 102 may be configured to receive the scan data 204A associated with the dentition 110 and generate a 3D model of the dentition 110 using the scan data 204A. Further, the processor 202 of the system 102 may be configured to generate anomaly data of the dentition 110 based on the 3D model of the dentition 110, and further generate interface data to output the first user interface associated with the dentition 110. The processor 202 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application-specific integrated circuit), an FPGA (field programmable gate array), a microcontroller unit (MCU), a hardware accelerator, a special-purpose computer chip, or the like. As such, in some embodiments, the processor 202 may include one or more processing cores configured to perform independently. A multi-core processor may enable multiprocessing within a single physical package. Additionally, or alternatively, the processor 202 may include one or more processors configured in tandem via the bus to enable independent execution of instructions, pipelining, and/or multithreading. Additionally, or alternatively, the processor 202 may include one or more processors capable of processing large volumes of workloads and operations to provide support for big data analysis. In an example embodiment, the processor 202 may be in communication with the memory 204 via a bus for passing information among components of the system 102.

For example, when the processor 202 may be embodied as an executor of software instructions, the instructions may specifically configure the processor 202 to perform the algorithms and/or operations described herein when the instructions are executed. However, in some cases, the processor 202 may be a processor-specific device (for example, a mobile terminal or a fixed computing device) configured to employ an embodiment of the present disclosure by further configuration of the processor 202 by instructions for performing the algorithms and/or operations described herein. The processor 202 may include, among other things, a clock, an arithmetic logic unit (ALU), and logic gates configured to support the operation of the processor 202. The network environment, such as 100 may be accessed using the communication interface 208 of the system 102. The communication interface 208 may provide an interface for accessing various features and data stored in the system 102.

In some embodiments, the processor 202 may be configured to provide Internet-of-Things (IoT) related capabilities to users of the system 102 disclosed herein. The IoT-related capabilities may in turn be used to provide smart solutions by providing real-time interface data generation, anomaly data generation, etc. by using the cloud-based systems for receiving scan data 204A and/or processing the scan data 204A. The I/O interface 206 may provide an interface for accessing various features and data stored in the system 102.

In an embodiment, the model generation module 202A may receive the scan data 204A of the dentition 110 that may include 2D images of the dentition 110 from various angles. Further, the model generation module 202A may use the ML module 202C to generate the 3D model data 204B for the 3D model of the dentition 110 based on the scan data 204A. The 3D model data 204B for the 3D model of the dentition 110 may be obtained alternatively by applying known 3D scanning principles such as triangulation or focus scanning techniques.

The processing module 202B of the processor 202 may be configured to analyse the scan data 204A. In an embodiment, based on the analysis, the processing module 202B may be configured to identify whether the scan data is sufficient to generate the 3D model correctly or not. In another embodiment, based on the analysis, the processing module 202B may be configured to identify whether the scan data and/or the 3D model has enough information to determine the anomaly data correctly. For example, in a case where scan data does not provide enough information to determine the anomaly data for a particular portion of the dentition 110. This may be a case when during the scan session the dental practitioner may not have used the scanning device 106 properly, which may have led to improper scanning of at least a portion of the dentition 110 and subsequently the images in the scan data do not have enough information to predict the anomaly data for the portion of the dentition 110 that was not properly scanned.

The ML module 202C of the processor 202 may be configured to execute ML model(s) 204C to carry out operations of the system 102. The ML module 202C may execute the ML model(s) 204C stored in the memory 204. In an example, the ML module 202C may execute a trained first ML model of the ML model(s) 204C for generating the 3D model data 204B for the 3D model of at least a portion of the dentition 110 using the scan data 204A. In an example, the scan data 204A may be input to the trained first ML model. The 3D model data 204B for the 3D model of at least a portion of the dentition 110 may be derived from the scan data 204A via known methods other than machine learning. The first ML model may be trained on historical scan data of dentitions of the plurality of patients to generate 3D models for each of one or more portions of the different dentitions. Subsequently, the trained first ML model is configured to generate the 3D model data 204B of at least the portion of the dentition 110 of the patient 112. Further, in another example, the ML module 202C of the processor 202 may execute a trained second ML model of the ML model(s) 204C for determining the anomaly data for at least the portion of the dentition 110 using the generated 3D model data 204B of the portion of the dentition 110. The second ML model may be trained on historical 3D models of dentitions of the plurality of patients to determine anomaly data for each of one or more portions of the different dentitions. Subsequently, the trained second ML model is configured to determine the anomaly data of at least the portion of the dentition 110 of the patient 112.

The output module 202D of the processor 202 may be configured to generate first interface data of at least a part of the dentition 110 based on the determined anomaly data associated with the dentition 110. For example, based on the output of the second ML model indicating that a first portion of the dentition 110 is affected by a dental anomaly DA1, the first interface data is generated. In such a case, the first interface data may include a user selectable interface element IE1 corresponding to the dental anomaly DA1. Also, one or more anomaly views corresponding to the dental anomaly DA1 may be generated and stored in the memory 204. For example, the anomaly data indicates that the first portion of the dentition 110 is affected by the anomaly DA1, and a second portion of the dentition 110 is affected by an anomaly DA2, then the anomaly views corresponding to both the anomalies DA1 and DA2 may be generated.

The memory 204 of the system 102 may be configured to store data including, but not limited to, the scan data 204A, the 3D model data 204B, the ML model(s) 204C, and other data 204D. The other data 204D may include, but is not limited to, generated 3D model(s) of the dentitions that have been observed in the past, report of the dentition 110 based on the anomaly data associated with the dentition 110, reports that may have been generated based on anomaly data for the dentitions that have been observed in the past, etc. The memory 204 may be non-transitory and may include, for example, one or more volatile and/or non-volatile memories. In other words, for example, the memory 204 may be an electronic storage device (for example, a computer-readable storage medium) comprising gates configured to store data (for example, bits) that may be retrievable by a machine (for example, a computing device like the processor 202). The memory 204 may be configured to store information, data, content, applications, instructions, or the like, for enabling the system 102 to carry out various functions in accordance with an example embodiment of the present disclosure. For example, the memory 204 may be configured to buffer input data for processing by the processor 202. As exemplarily illustrated in FIG. 2, the memory 204 may be configured to store instructions for execution by the processor 202. As such, whether configured by hardware or software methods, or by a combination thereof, the processor 202 may represent an entity (for example, physically embodied in circuitry) capable of performing operations according to an embodiment of the present disclosure while configured accordingly. Thus, for example, when the processor 202 is embodied as an ASIC, FPGA, or the like, the processor 202 may be specifically configured hardware for conducting the operations described herein.

The scan data 204A may include at least the 2D images of the dentitions, for example, the dentition 110 of the patient 112, and dentitions of other patients for whom the dentitions have been scanned in the past, and scan data received by the system 102.

The 3D model data 204B may include the 3D model(s) generated for the dentition 110 for which the scan data 204A has been received. For example, the 3D model(s) generated for the dentition 110 of the patient 112 may be included in the 3D model data 204B.

The ML model(s) 204C may include trained ML models that may be used by various components of the processor 202, such as the processing module 202B, the model generation module 202A, the ML module 202C, etc. for performing various operations that require implementation of machine learning models. For example, the ML models 204C may include the trained first ML model for generating the 3D model data 204B indicating a 3D model of a portion of the dentition 110 using the scan data 204A. For example, the ML models 204C may also include the trained second ML model for determining anomaly data for the portion of the dentition 110 using the generated 3D model data 204B of the portion of the dentition 110.

In an exemplary embodiment, the machine learning model 204C may be used for various tasks such as, but not limited to, classification, regression, pattern recognition, and decision-making. Examples of the ML model(s) 204C may include, but are not limited to, an artificial neural network (ANN), a deep neural network (DNN), a convolutional neural network (CNN), a fully connected neural network, and/or a combination of such networks.

In some example embodiments, the I/O interface 206 may communicate with the system 102 and display output of the system 102. In an embodiment, the I/O device 206 may include the display device 206A. Examples of the display device 206A may include, but are not limited to, an LED screen, an LCD screen, an AMOLED screen, etc. The display device 206A may be utilized by the system 102 to display user-selectable interface elements of the first user interface associated with the dentition 110.

As such, the I/O interface 206 may include, in some embodiments, may also include a keyboard, a mouse, a joystick, a touch screen, touch areas, soft keys, one or more microphones, a plurality of speakers, or other input/output mechanisms. In one embodiment, the system 102 may include a user interface circuitry configured to control at least some functions of one or more I/O interface elements such as the display device 206A and, in some embodiments, a plurality of speakers, a ringer, one or more microphones and/or the like. The processor 202 and/or I/O interface circuitry may be configured to control one or more functions of one or more I/O interface elements through computer program instructions (for example, software and/or firmware) stored on the memory 204 and accessible to the processor 202.

The communication interface 208 may include an input interface and output interface for supporting communications to and from the system 102 or any other component with which the system 102 may communicate. The communication interface 208 may be any means such as a device or circuitry embodied in either hardware or a combination of hardware and software that is configured to receive and/or transmit data to/from a communications device in communication with the system 102. In this regard, the communication interface 208 may include, for example, an antenna (or multiple antennae) and supporting hardware and/or software for enabling communications with a wireless communication network. Additionally, or alternatively, the communication interface 208 may include the circuitry for interacting with the antenna(s) to cause transmission of signals via the antenna(s) or to handle receipt of signals received via the antenna(s). In some environments, the communication interface 208 may alternatively or additionally support wired communication. As such, for example, the communication interface 208 may include a communication modem and/or other hardware and/or software for supporting communication via cable, digital subscriber line (DSL), universal serial bus (USB), or other mechanisms. In some embodiments, the communication interface 208 may enable communication with a cloud-based network to enable deep learning, such as using the ML model(s) 204C (that may be hosted on the cloud-based network).

FIG. 3 illustrates a block diagram 300 depicting exemplary operations for displaying a first user interface associated with the dentition 110 of the patient 112, in accordance with an embodiment of the disclosure. FIG. 3 is explained in conjunction with elements from FIG. 1 and FIG. 2. The operations may start at 302.

At 302, scan data reception operation may be executed. In an embodiment, the system 102 may be configured to receive the scan data 204A. In an embodiment, the system 102 may receive the scan data 204A from a source such as, but not limited to, the scanning device 106, the memory 204, the database 108, etc. The scan data 204A may include 2D images of the dentition 110 of the patient 112. For example, a dental practitioner may scan the dentition 110 of the patient 112 using the scanning device 106 to obtain the scan data 204A. The scanning device 106 may include, but not limited to, a handheld intraoral scanner.

In an embodiment, the 2D images of the dentition 110 may be of high-resolution, providing details of the tooth surfaces, including the enamel, dentin, and any existing cavities or lesions. The 2D images may include coloured images to enhance the visualization of the scanned portion of the dentition 110. The 2D images may provide a limited sense of depth and contour according to the ability of the scanning device 106 to capture the surface topography of the teeth and surrounding tissues within the dentition 110.

At 304, a 3D model generation operation may be executed. In an embodiment, the system 102 may be configured to generate a 3D model of the dentition 110 based on the scan data 204A. In an example, the model generation module 202A may be configured to use the ML model(s) 204C for generating the 3D model based on the received scan data 204A. In an embodiment, the ML model(s) 204C may include a first ML model. The first ML model may be configured to analyse the scan data 204A and generate the 3D model of the dentition 110 based on the analysis of the scan data 204A. In an embodiment, the ML module 202C of the processor 202 may be configured to implement the first ML model. The first ML model may be trained on several 2D images of various dentitions associated with various patients to generate 3D models. The training may also include human inputs such as, labelling of the input data for training the first ML model. Generating the 3D model of the dentition 110 based on the scan data 204A may alternatively comprise "stitching" together individual sub-scans comprised of 2D images of the scan data 204A until the complete 3D model is generated.

In an exemplary embodiment, once the system 102 may receive the scan data 204A from the source, the system 102 may provide the received scan data as input to the first ML model for the generation of the 3D model data 204B from the scan data 204A.

At 306, an anomaly data generation operation may be executed. In an embodiment, the system 102 may be configured to generate the anomaly data associated with the dentition 110. In an embodiment, the system 102 may be configured to receive output from the model generation module 202A in the form of a 3D model, such as, the 3D model data 204B. The 3D model data 204B may include a 3D model of the dentition 110. Further, based on the received 3D model data 204B, the system 102 may be configured to determine the anomaly data associated with the dentition 110. The anomaly data may pertain to one or more dental anomalies from a plurality of dental anomalies. The plurality of dental anomalies may include, but is not limited to, a surface caries anomaly, an occlusal caries anomaly, a proximal caries anomaly, a tooth wear anomaly, a gingival recession anomaly, a gingivitis anomaly, and/or a plaque anomaly. Subsequently, the anomaly data may indicate information associated with one or more dental anomalies from the plurality of dental anomalies. These one or more dental anomalies may be present or occurring in the dentition 110 of the patient 112.

In an embodiment, the system 102 may use the ML module 202C of the processor 202 to determine the anomaly data associated with the 3D model data 204B. The ML module 202C may execute a trained second ML model for determining the anomaly data for the generated 3D model of the dentition 110. The second ML model may be trained on historical 3D models of dentitions of the plurality of patients to determine anomaly data for each of one or more portions of the different dentitions. Subsequently, the trained second ML model is configured to determine the anomaly data of the dentition 110 of the patient 112. Exemplary details regarding the determination of anomaly data using the second ML model are provided in conjunction with, for example, FIG. 15A and FIG. 15B. Pertinently, a portion of the dentition 110 may refer to a portion of the 3D model that can be used to determine the anomaly data for the dentition 110.

In an embodiment, the anomaly data indicates occurrence of one or more dental anomalies among the plurality of dental anomalies in the dentition 110 of the patient 112. For example, the anomaly data may indicate that the first portion of the dentition 110 may be affected by a surface caries anomaly, a second portion of the dentition 110 may be affected by a gingival recession anomaly, and a third portion of the dentition 110 may be affected by a plaque anomaly. In an example, the anomaly data may include data corresponding to the surface caries anomaly, data corresponding to the gingival recession anomaly, and data corresponding to the plaque anomaly. It may be noted that a portion of dentition 110, say the first portion of the dentition affected by the surface caries anomaly may correspond to one or more teeth of the dentition 110 or to one or more tooth surfaces such as occlusal, buccal, labial and/or lingual surface. In case the first portion affected with the surface caries anomaly corresponds to two or more teeth of the patient, such two or more teeth may be located across various locations, such as in the upper jaw and/or in the lower jaw of the patient 112. To this end, the portion of the dentition 110 affected with a dental anomaly may correspond to a continuous area affected with the dental anomaly, or discontinuous areas spread across the oral cavity of the patient 112 where such discontinuous areas are affected with the dental anomaly.

In an embodiment of the disclosure, the anomaly data may indicate an anomaly score associated with at least one dental anomaly of the plurality of dental anomalies. The anomaly score for a dental anomaly may indicate an extent to which the portion of the dentition 110 may be affected. For example, where the anomaly data includes the data corresponding to the occurrence of the surface caries anomaly in the dentition 110, the anomaly score may indicate a number of teeth and/or a percentage of the dentition 110 affected by the surface caries anomaly. The anomaly score may include a numerical value and/or a rating indicating the number of affected teeth or the percentage of the dentition 110 affected by the surface caries anomaly. In certain cases, the anomaly score may also indicate a level of severity or an extent to which surface caries anomaly has affected the dentition 110.

For example, 3 teeth (namely, T1, T2, and T3) may be affected with surface caries anomaly, 2 teeth (namely, T4 and T5) may be affected with gingival recession anomaly, and one tooth (namely T6) may be affected with plaque anomaly. In such case, a first anomaly score for the surface caries anomaly may be higher than a second anomaly score for the gingival recession anomaly as a number of teeth affected with surface caries anomaly is higher than a number of teeth affected with gingival recession anomaly. In an example, the extent to which the teeth are affected by the dental anomaly may also be a factor in generating the anomaly score. For example, 3 teeth (namely, T1, T2, and T3) may be affected by the surface caries anomaly, while 2 teeth (namely, T4 and T5) may be affected by the gingival recession anomaly, but the extent of the gingival recession anomaly is very high as compared to the extent of the surface caries anomaly. In such case, the second anomaly score of the gingival recession anomaly may be higher than the first anomaly score of the surface caries anomaly.

In an embodiment, the anomaly score for a particular dental anomaly may include percentage of an area of tooth or teeth, or the number of tooth or teeth affected by the dental anomaly among the total number of teeth present in the scan data 204A. For example, the scan data 204A includes a total number of 32 teeth of which 16 teeth are affected by plaque. Then the anomaly data may indicate a calculated percentage of the teeth affected by plaque, i.e., 50% in case 16 teeth out of a total number of 32 teeth are affected by plaque. Further, the anomaly data may also include severity overview information indicating severity extent of the dental anomaly and a category for the severity extent of the dental anomaly. The severity overview information for a dental anomaly may include information such as, but not limited to, a category of the severity extent of the dental anomaly, a number and/or position of each of the teeth affected by the dental anomaly, a percentage of examined teeth affected by the dental anomaly, severity extent of each of the teeth affected by the dental anomaly, etc. Also, such above information may be included in the severity overview information and shown against name of the dental anomaly, and/or the first visual representation and/or the second visual representation of the dental anomaly. Examples of categories for the severity extent of the dental anomalies may include, but are not limited to, 'severe', 'moderate', and 'initial'. For example, 0-10% of teeth affected by a dental anomaly may be categorized as initial, 11-25% of teeth affected by the dental anomaly may be categorized as moderate, and 26-100% of teeth affected by the dental anomaly may be categorized as severe.

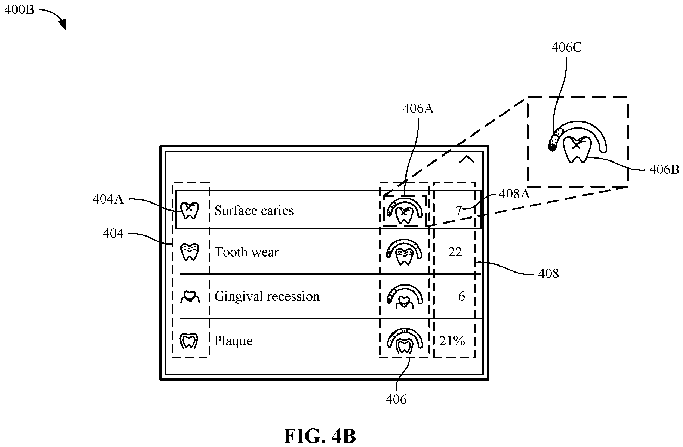

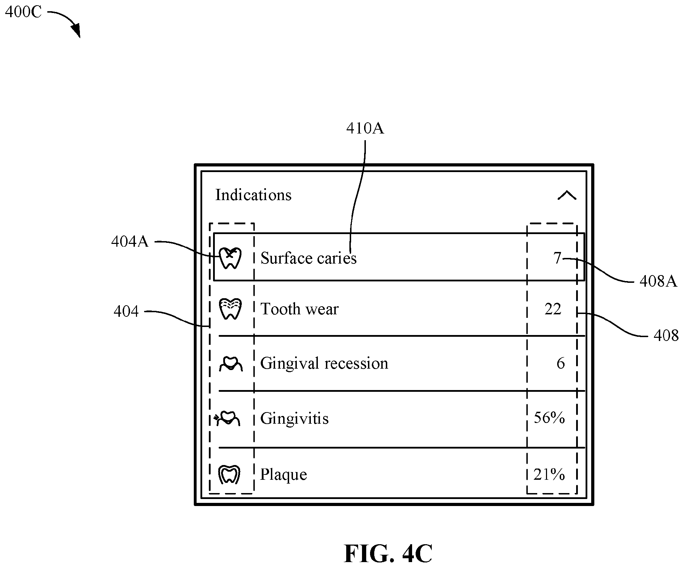

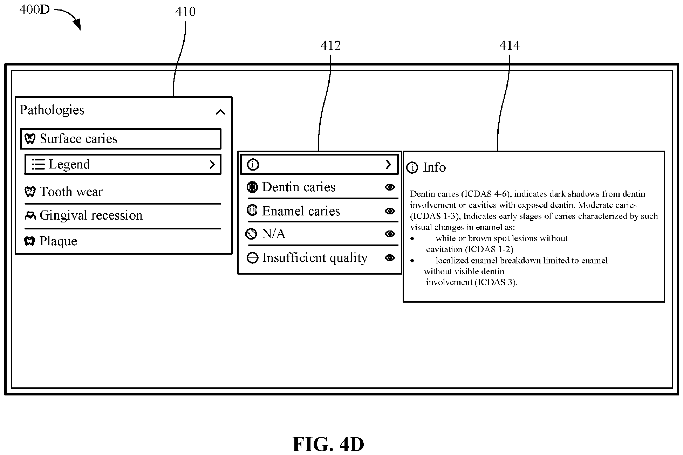

At 308, an interface data generation operation may be executed. In an embodiment, the system 102 may be configured to generate first interface data 308A based on the 3D model and/or the anomaly data of the dentition 110. The first interface data 308A may be associated with a first user interface 310A. The first user interface 310A may include one or more user-selectable interface elements. Variations of presentation of the one or more user-selectable interface elements of the first user interface 310A are provided in conjunction with FIG. 4A, FIG. 4B, FIG. 4C, and FIG. 4D. Each of the one or more user-selectable interface elements may be selectable to generate an anomaly view of the dentition 110, such that the anomaly view indicates a corresponding dental anomaly of the plurality of dental anomalies within the 3D model.

In an embodiment, the first user interface 310A may include the one or more user-selectable interface elements. The one or more user-selectable interface elements may correspond to the one or more dental anomalies that are detected in the dentition 110 based on the 3D model data of the dentition 110. For example, the 3D model data 204B indicates that 3 teeth (namely, T1, T2, and T3) are affected with the surface caries anomaly, 2 teeth (namely, T4 and T5) are affected with the gingival recession anomaly, and one tooth (namely T6) is affected with the plaque anomaly. In such case, the one or more user-selectable interface elements include an element corresponding to the surface caries anomaly, an element corresponding to the gingival recession anomaly, and an element corresponding to the plaque anomaly.

In an embodiment, the one or more user-selectable interface elements may be present as list items, horizontal menu tabs, vertical menu tabs, and any such form that may be suitable for presentation to the user via the first user interface 310A. Exemplary variations of the first user interface 310A that may be displayed via the display device 206A of the system 102 are provided in conjunction with FIG. 4A, FIG. 4B, FIG. 4C, FIG. 4D, FIG. 5A, FIG. 5B, FIG. 5C, FIG. 6A, and FIG. 6B. In an embodiment, the first interface data 308A may include various derivations of the anomaly data for presenting the anomaly data in a user-friendly manner via the first user interface 310A. In an example, the first interface data 308A may include derivation of the anomaly data such that anomaly data for each of the one or more anomalies present in the dentition 110 is presented through the one or more user-selectable interface elements. In an example, the first interface data 308A may include at least a portion of the anomaly data to be presented in association with each of the one or more user-selectable interface elements within the first user interface 310A.

In an embodiment, the system 102 may be configured to receive first visual representation data associated with the plurality of dental anomalies. In an embodiment, the first visual representation data may include a plurality of first visual representations. Each first visual representation of the plurality of first visual representations is associated with a dental anomaly of the plurality of dental anomalies. In an example, a first visual representation associated with a dental anomaly may correspond to, but is not limited to, an icon of the dental anomaly, a logo of the dental anomaly, a symbol of the dental anomaly, a text format associated with the dental anomaly, and/or a combination thereof to indicate the dental anomaly among the plurality of dental anomalies. For example, each of the plurality of first visual representations may be associated with a dental anomaly among the plurality of dental anomalies, and no two dental anomalies may be associated with a same first visual representation. Therefore, the plurality of first visual representations may serve as visual identifiers for various dental anomalies.

Further, the system 102 may be configured to generate second visual representation data associated with the plurality of dental anomalies based on the anomaly data. The second visual representation data may indicate a plurality of second visual representations of the anomaly score of the corresponding dental anomaly of the plurality of dental anomalies.

In an embodiment, the system 102 is configured to determine an anomaly score associated with a specific dental anomaly of the plurality of dental anomalies based on the anomaly data associated with the specific dental anomaly. Thereafter, the system 102 is configured to generate categorisation data associated with the specific second visual representation for the specific dental anomaly based on the anomaly score. The categorization data may indicate for example, but not limited to, one or more division areas of the specific second visual representation, a severity category from a plurality of severity categories for each of the one or more division areas, and/or a visual indicator from a plurality of visual indicators for each of the one or more division areas. Further, the system 102 is configured to generate the second visual representation data associated with the specific dental anomaly based on the categorisation data. For example, for the specific dental anomaly, say, tooth wear anomaly, the anomaly data indicates that a total of 16 out of 32 teeth are affected by the tooth wear anomaly. For example, the number of teeth severely affected by tooth wear anomaly in the dentition 110 is 4, and the number of teeth moderately affected by tooth wear anomaly in the dentition 110 is 12. Assuming, for example, there are 32 division areas in the second visual representation, representing 32 teeth. Moreover, different severity categories may be associated with different visual indicators, say the severity category 'severe' is represented by a visual indicator corresponding to red colour, and the severity category 'moderate' is represented by a visual indicator corresponding to orange colour. Then, for the dentition 110 for tooth wear anomaly, the second visual representation may show 16 out of 32 division areas as coloured indicating affected teeth, and a visual indicator of 4 out of 16 coloured division areas may be in red colour indicating severity category 'severe', while a visual indicator of 12 out of 16 coloured division areas may be in orange colour indicating severity category 'moderate'. An advantage of having the second visual representation generated in the described manner is that two types of information are conveyed simultaneously, both the information on number of affected teeth and the information on severity of the dental anomaly. This type of information may guide the user to assess whether a dental treatment is required. Alternatively, the dental treatment may be automatically proposed based on the anomaly data, either on the number of teeth affected, the severity of the dental anomaly or the combination of both.

In an embodiment, the second visual representation data of the specific second visual representation may include a graph, an arch, a bar, a circular representation, a semi-circular representation, a mathematical value, a number, a text, a colour, a symbol, and/or a combination thereof to indicate an anomaly score of the specific dental anomaly of the plurality of dental anomalies. For example, the specific second visual representation may comprise an arch of different colours in a semi-circular design. The arch may correspond to a scale indicating a percentage of the number of teeth in the dentition 110 being affected by the specific dental anomaly. Also, the arch may be coloured using different colours to provide more information, such as severity. For example, the dentition 110 includes a total of 32 teeth of which 16 teeth are affected by the gingival recession anomaly. Further, out of the 16 affected teeth, 8 teeth are severely affected, and 8 teeth are moderately affected. Also, for example, the severe category is indicated in red colour, and moderate category is indicated in yellow colour. In such case, the arch may extend to 50% in colour where 25% of the arch is shown in red colour and the other 25% of the arch is shown in yellow colour. In this way, both the severity and the number of teeth affected are presented assisting the user to prioritize inspecting certain anomalies over other, by clinical significance. Specifically, to assess the clinical significance of an anomaly present, the information on number of teeth affected and information on severity may be required. Furthermore, the arch allows easy tracking of changes in severity distribution of the anomalies by the user. Additionally, such created second visual representation serves as aid in patient education and patient communication, as aid in-clinic comuunication such as communication between a hygienist and a dentist, as a documentaion aid capable of providing legal support with respect to insurance and/or reimbursement approvals.

To this end, the system 102 is configured to generate the first interface data based on the first visual representation data and/or the second visual representation data. Further, each of the one or more user-selectable interface elements may indicate at least one of: a first visual representation of the plurality of first visual representations, or a second visual representation of the plurality of second visual representations, based on corresponding dental anomaly of the plurality of dental anomalies. Continuing with the above example where 50% of teeth are affected with the gingival recession anomaly, the coloured arch showing the 50% percentage of teeth affected may be shown above a symbol that represents the gingival recession anomaly. For example, the symbol is the first visual representation corresponding to the gingival recession anomaly. Summarily, each of the plurality of first visual representations indicates a symbol associated with the corresponding dental anomaly of the plurality of dental anomalies, and each of the plurality of second visual representations indicates a scale for the anomaly score of the corresponding dental anomaly of the plurality of dental anomalies.

At 310, a first user interface output operation may be executed. In an embodiment, the system 102 may be configured to output a first user interface based on the first interface data. In an embodiment, during the first user interface output operation 310, the system 102 may be configured to control the display device 206A to display the first user interface 310A. The first user interface 310A may indicate the first interface data 308A in a form presentable to the user. The first user interface 310A may include one or more user-selectable interface elements that may be clicked or touched or interacted with for the presentation of anomaly data associated with the dentition 110 of the patient 112 in a user-friendly manner. In an example, the first user interface 310A is output or rendered using the display device 206A to enable the user to interact with the one or more user-selectable interface elements. The display device 206A may include, but not limited to, a screen, a monitor, a touchpad, a Cathode Ray Tube (CRT) monitor, a plasma display, light emitting diode (LED) display, an LCD display, etc. Some exemplary variations of the first user interface 310A as displayed on the display device 206A are provided with reference to FIG. 4A, FIG. 4B, FIG. 4C, FIG. 4D, FIG. 5A, FIG. 5B, FIG. 5C, FIG. 6A, and FIG. 6B.

In an embodiment, the system 102 may control/cause the display device 206A to display the first user interface 310A. Consequently, the one or more user-selectable interface elements may be displayed on the display device 206A. Reiterating from above, the one or more user-selectable interface elements may correspond to the one or more dental anomalies that are detected in the dentition 110 based on the 3D model data 204B of the dentition 110. For example, where the anomaly data indicates a first portion of the dentition 110 is affected by the surface caries anomaly, a second portion of the dentition 110 is affected by the gingival recession anomaly, and a third portion of the dentition 110 is affected with the plaque anomaly, the one or more user-selectable interface elements may include a first interface element corresponding to the surface caries anomaly, a second interface element corresponding to the gingival recession anomaly, and a third interface element corresponding to the plaque anomaly. In an example, each of the first interface element, the second interface element, and the third interface element may include a first visual representation, such as a symbol of corresponding dental anomaly. Moreover, each of the first interface element, the second interface element, and the third interface element may include a second visual representation, such as a scale corresponding to an anomaly score of the corresponding dental anomaly. In certain cases, the each of the first interface element, the second interface element, and the third interface element may also include additional representations, such as textual data indicating the corresponding dental anomaly, a type of representation, such as colour or gradient, associated with the corresponding dental anomaly, and so forth.

In an embodiment, the system 102 may be configured to receive a user selection of the first dental anomaly of the plurality of dental anomalies after outputting or displaying the first user interface 310A via the display device 206A. The user selection may be made by one of clicking action, a touch action, a swipe action, a motion gesture, or any such action that may be suitably made by the user to provide an input regarding user selection at the first user interface 310A. Notably, since the one or more user-selectable interface elements correspond to the one or more dental anomalies that are determined in the dentition 110, the user selection of one of the user-selectable interface elements corresponds to the selection of a dental anomaly among the one or more dental anomalies that are determined in the dentition 110. Thus, the system 102 may receive the user selection of the first dental anomaly.

Further, the system 102 may be configured to generate a first anomaly view of the dentition 110. The first anomaly view may include at least the first portion of the 3D model. In an example, at least the first portion of the 3D model indicates an occurrence of the first dental anomaly in the dentition 110 of the patient 112. For example, the one or more user-selectable interface elements may correspond to dental anomalies, such as the surface caries anomaly, the gingival recession anomaly, and/or the plaque anomaly. Say, the system 102 receives a user selection of the surface caries anomaly. Upon receiving the user selection of the surface caries anomaly, the system 102 is configured to generate a first anomaly view of the dentition 110 where the first anomaly view highlights a portion of the dentition 110 that is affected by the surface caries anomaly, i.e., a first portion of the 3D model that is affected by the surface caries anomaly. Here, the first portion refers to one or more teeth or the areas of the one or more teeth that are affected by the corresponding dental anomaly, e.g., the surface caries anomaly. For example, when an anomaly view highlights a portion of the dentition 110 that is affected by the plaque anomaly, only certain areas of teeth in the dentition 110 may be highlighted in the corresponding anomaly view to indicate where plaque is deposited. In an embodiment, the first anomaly view may highlight the first portion in various ways including, but not limited to, adding a colour overlay layer on the 3D model, adding a pattern view layer on the 3D model, outlining the first portion in bold or a different colour, and so forth.

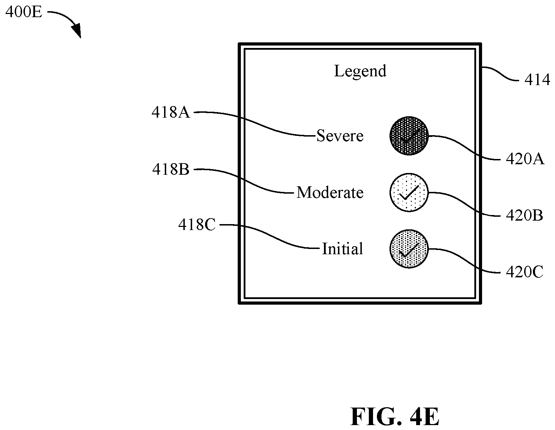

In an embodiment, the system 102 may cause/control the display device 206A to output or display the first anomaly view, such that the first anomaly view is overlaid on the first user interface 310A. In an example, the first anomaly view is shown on the first user interface 310A as a part of the first user interface 310A. For example, the first anomaly view may occupy a portion of the display device 206A while the first user interface 310A is displayed on the display device 206A. Also, according to the anomaly view and the first portion highlighted in the anomaly view of the dentition 110, the first user interface 310A may include a legend information. An exemplary legend information is illustrated in conjunction with, for example, FIG. 4E. The legend information may provide information regarding significance of each of the plurality of visual indicators, severity extent of the dental anomaly in various portions of the dentition 110, and so forth. For example, where the severity extent is categorized as 'severe', 'moderate', and 'initial', and the anomaly view includes a colour overlay layer to highlight the first portion, the legend information may contain the corresponding colour (that is present in the colour overlay layer) indicating the type of severity extent of the dental anomaly. For example, severe category is indicated by red colour, moderate category is indicated by orange colour, and initial category is indicated by yellow colour, and also the tooth T1 is affected by the surface caries anomaly severely, teeth T2 and T3 are moderately affected by the surface caries anomaly, and certain portion of tooth T4 is initially affected by the surface caries anomaly. Then, the tooth T1 may be highlighted in red colour, teeth T2 and T3 may be highlighted in orange colour, and the certain portion of the tooth T4 may be highlighted in yellow colour. Such mapping between colour and severity type may be made apparent to the user via the legend information.

In an embodiment, the system 102 may be configured to generate report data based on the anomaly data and the first interface data. The report data indicates at least one of: a health status of the dentition 110, and/or a treatment for at least one dental anomaly of the plurality of dental anomalies. The system 102 may further be configured to generate and store a report for the dentition 110 of the patient 112, in the memory 204 based on the report data. Also, in an embodiment, one or more versions of the report may be generated by the system 102. For example, a first version of the report may provide detailed information useful for a dental practitioner, a second version of the report may provide a simplified information useful for the patient 112. Examples of the report data may include, but are not limited to, a name and contact information of the user, an identity information of the patient, 2D images of the scan data 204A of the dentition 110, images of 3D model generated for the dentition 110, trend information associated with the plurality of dental anomalies based on historical dental examinations of the dentition 110, detailed information about the teeth affected with one or more dental anomalies in the dentition 110 (for example, the detailed information may include the standard position (according to universal numbering system for teeth) of each teeth that may be affected by each of the plurality of dental anomalies, and severity extent of the each of the plurality of dental anomalies with which the teeth may be affected), a condition information of one or more teeth in the dentition 110, and a diagnose summary of one or more teeth in one or more portions of dentition 110, one or more screenshots of the first user interface 310A.

In an embodiment, the system 102 is configured to determine a timestamp associated with the generation of the anomaly data, the first interface data 308A, and/or the first user interface 310A. The timestamp may indicate at least one of a date, or a combination of date and time of generation of the anomaly data, the first interface data 308A, and/or the first user interface 310A associated with the dentition 110. Alternatively, the timestamp may indicate a time (i.e., the combination of date and time) of generation of the 3D model data 204B associated with the dentition 110, or the timestamp may indicate a time of reception of the scan data 204A associated with the dentition 110, or the timestamp may indicate a time of generation of the first interface data 308A associated with the dentition 110. Using the timestamp, data associated with the dentition 110, generated or received by the system 102 may be accessed. The data associated with the dentition 110 may include, but is not limited to, the scan data 204A, the 3D model data 204B, the other data 204D, the first interface data 308A, the second interface data, and the anomaly view data.

FIGs. 4A-4D show various exemplary variation of the first user interface 310A associated with the dentition 110 that may be displayed via the display device 206A of the computer system 102, in accordance with various embodiments of the disclosure. FIGs. 4A-4D are explained in conjunction with elements from FIG. 1, FIG. 2, and FIG. 3.

Referring to FIG. 4A, a diagram 400A of a first exemplary variation of the presentation of the one or more user-selectable interface elements 400 of the first user interface 310A associated with the dentition 110 is shown. In 400A, a list of dental anomalies from the plurality of dental anomalies is shown as the user-selectable interface elements 402. As shown, the user-selectable interface elements 402 include a first interface element 402A corresponding to the surface caries anomaly, a second interface element 402B corresponding to the tooth wear anomaly, a third interface element 402C corresponding to the gingival recession anomaly, and a fourth interface element 402D corresponding to the plaque anomaly. Notably, the list of one or more dental anomalies as shown in 402 are user-selectable interface elements. Also, each interface element of each of the one or more dental anomalies includes a corresponding symbol or the corresponding first visual representation. In an example, the first visual representation for a dental anomaly along with corresponding textual data in an interface element are collectively shown as 404. For example, the first interface element 402A for the surface caries anomaly includes a first visual representation 404A depicting a symbol indicating the surface caries anomaly. It would be prudent to infer from the above discussion with reference to FIG. 3 that the dentition 110 corresponding to the diagram 400A has a first portion of teeth affected with the surface caries anomaly, a second portion of the teeth affected with the tooth wear anomaly, a third portion of the teeth affected with the gingival recession anomaly, and a fourth portion of the teeth affected with the plaque anomaly. Also, the first, second, third, and fourth portions of teeth may include the same or a different portion of teeth.

Referring to FIG. 4B, a diagram 400B of a second exemplary variation of the one or more user-selectable interface elements of the first user interface 310A associated with the dentition 110 is shown. In 400B, the presentation of the one or more user-selectable interface elements of the first user interface 310A may include severity overview information for each of the plurality of dental anomalies. The severity overview information may include the first visual representation and the second visual representation for each of the plurality of dental anomalies that are collectively shown in 406. For example, the first visual representation and the second visual representation in the first interface element 402A for the surface caries anomaly is shown in 406A. In the enlarged view of 406A, the first visual representation is shown as 406B, and the second visual representation is shown as 406C. The first visual representation 406B may include the symbol of the surface caries anomaly. The second visual representation 406C may include a graph, a scale, or gauge/arch which further provides information on the severity extent of the dentition 110 affected by the surface caries anomaly. Also, the second visual representation 406C indicating the anomaly data associated with the surface caries anomaly may have a semi-circular design, a linear design, a circular design, etc. For example, a semi-circular design of an arch may represent overall severity extent of the dental anomaly with which the teeth in the dentition 110 may be affected. In another example, a circular design of the arch may represent separately the severity extent of teeth affected in the lower jaw of dentition 110 and upper jaw of dentition 110. The arch of the second visual representation 406C may indicate a scale from 0 to 100% teeth in the dentition 110 being affected. For example, the second visual representation 406C may include different portions of the arch in different colours to represent different categories of severity extent of the dental anomaly, or the arch may include different portions of the arch in different patterns to represent different categories of the severity extent of the dental anomaly. In an embodiment, the severity overview information may further include text information 408 associated with the severity extent of each of the plurality of dental anomalies. For example, the text information 408 associated with the severity extent of the surface caries anomaly in the first user-selectable interface element 402A is shown as text information 408A. For example, the text information 408A may include a numerical value '7' indicating that 7 teeth of the total number of scanned teeth in the dentition 110 are affected by the surface caries anomaly. The first visual representations shown as 404 may optionally be present in the diagram 400B.

Referring to FIG. 4C, a diagram 400C of a third exemplary variation of the presentation of the one or more user-selectable interface elements of the first user interface 310A associated with the dentition 110 is shown. In 400C, the presentation of the one or more user-selectable interface elements of the first user interface 310A may include the severity overview information for each of the plurality of dental anomalies, where the severity overview information includes the text information 408 associated with the severity extent of each of the plurality of dental anomalies, but the severity overview information does not include the first visual representation (such as symbols, logo or icon) and the second visual representation (such as scale or arch) for any of the plurality of dental anomalies.

Referring to FIG. 4D, a diagram 400D of a fourth exemplary variation of the presentation of the one or more user-selectable interface elements of the first user interface 310A associated with the dentition 110 is shown. In 400D, the presentation of the one or more user-selectable interface elements of the first user interface 310A includes a list of interface elements 410 corresponding to the plurality of dental anomalies and a legend information tab 412. On selecting an interface element from the list of interface elements 410 corresponding to a specific dental anomaly among the plurality of dental anomalies, a particular legend information window 412 is shown. The legend information window 412 may include various elements such as, but not limited to, an icon for showing general information related to the specific dental anomaly that is selected, and key information indicating the severity overview information of the selected specific dental anomaly. In an embodiment, the general information is shown based on the selection of an icon included in the legend information window 412.

Referring to FIG. 4E, a diagram 400E of an exemplary variation of legend information 414 associated with the first user interface 310A associated with the dentition 110 is shown. As shown in 400E, the legend information 414 may correspond to the severity overview information that may be shown with the first user interface 310A. The legend information 414 may provide an information regarding severity extent of the dental anomaly. For example, where the severity extent is categorized as 'severe', 'moderate', and 'initial', the legend information 414 may include the severity extent of the dental anomaly and the corresponding colour/pattern used to show the corresponding severity extent. For example, the severity extent may be shown as severe 418A and the colour/pattern 420A may be used to depict or show the severe 418A severity extent in the first user interface 310A and/or an anomaly view. Similarly, colour/pattern 420B may be used to show the severity extent for moderate 418B, and colour/pattern 420C may be used to show the severity extent for initial 418C.

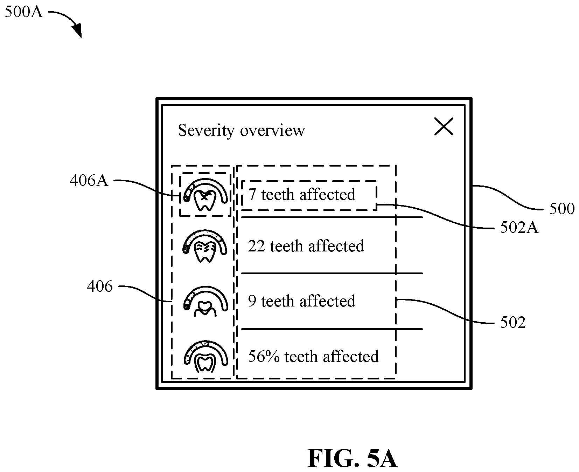

FIG. 5A shows a first exemplary variation of severity overview information associated with the first user interface 310A associated with the dentition 110, in accordance with an embodiment of the disclosure. Referring to FIG. 5A, a diagram 500A of the first exemplary variation of the severity overview information 500 associated with the first user interface 310A associated with the dentition 110 is shown. FIG. 5A may be explained in conjunction with elements from FIG. 1, FIG. 2, FIG. 3, and FIG. 4A - FIG. 4E. The first visual representations and the second visual representations of the plurality of dental anomalies are shown in 406, and anomaly occurrence information associated with each of the plurality of dental anomalies is shown in 502. For example, for a particular dental anomaly, say the surface caries anomaly, among the plurality of dental anomalies, the first visual representation, e.g., a symbol of the surface caries anomaly, and the second visual representation, e.g., a scale or an arch indicating the percentage of teeth affected and/or severity of affected teeth, are shown within 406A. Further, the anomaly occurrence information for the particular dental anomaly indicating a number of teeth affected with the surface caries anomaly is shown in 502A.

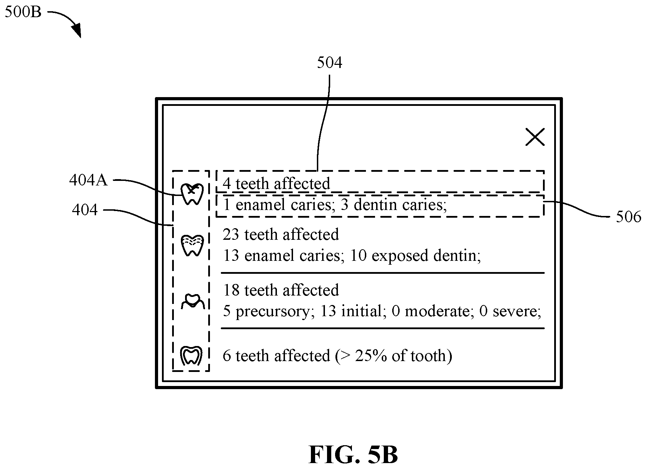

FIG. 5B shows a second exemplary variation of the severity overview information associated with the first user interface 310A, in accordance with an embodiment of the disclosure. Referring to FIG. 5B, a diagram 500B of the second exemplary variation of the severity overview information associated with the first user interface 310A is shown. FIG. 5B may be explained in conjunction with elements from FIG. 1, FIG. 2, FIG. 3, FIG. 4A- FIG. 4E, and FIG. 5A. In 500B, the first visual representation for each dental anomaly is collectively shown in 404 and a corresponding anomaly occurrence information is shown as text information against each of the first visual representations 404 for the plurality of dental anomalies occurring in the dentition 110. For example, the severity overview information for a particular dental anomaly, say the surface caries anomaly may be depicted or shown using a first visual representation 404A, anomaly occurrence information 504, and additional textual anomaly information 506. The anomaly occurrence information 504 may include an overall number of affected teeth with the surface caries anomaly, and the additional textual anomaly information 506 further provides bifurcation of the surface caries anomaly indicating types of the surface caries anomaly present in the dentition 110. In certain cases, the additional textual anomaly information 506 may also indicate the severity extent of a particular dental anomaly. In such a case, for example, a number of teeth severely affected with the particular dental anomaly, a number of teeth moderately affected with the particular dental anomaly, and/or a number of teeth initially affected with the particular dental anomaly may be provided.

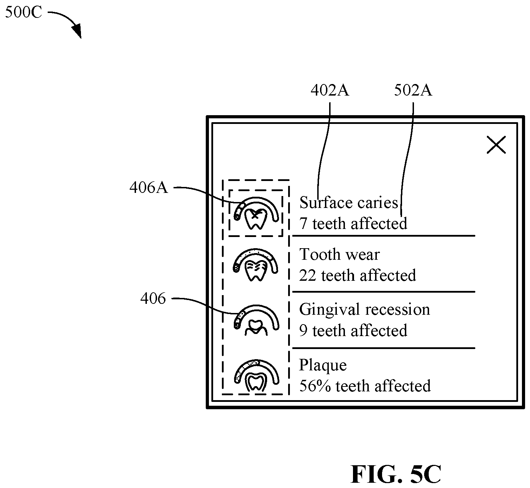

FIG. 5C shows a third exemplary variation of the severity overview information associated with the first user interface 310A, in accordance with an embodiment of the disclosure. Referring to FIG. 5C, a diagram 500C of the third exemplary variation of the severity overview information associated with the first user interface 310A is shown. FIG. 5C may be explained in conjunction with elements from FIG. 1, FIG. 2, FIG. 3, FIG. 4A-FIG. 4E, and FIG. 5A-FIG. 5B. In 500C, the first visual representation and the second visual representation are shown in 406. Further, each of the first visual representation and the second visual representation is shown against the name of the corresponding dental anomaly and the anomaly occurrence information associated with the dentition 110 for the dental anomaly. For example, a first interface element associated with a specific dental anomaly may include the first visual representation and the second visual representation (collectively illustrated as 406A), a name, e.g., "surface caries" of the specific dental anomaly, and occurrence information 502 for the surface caries anomaly.

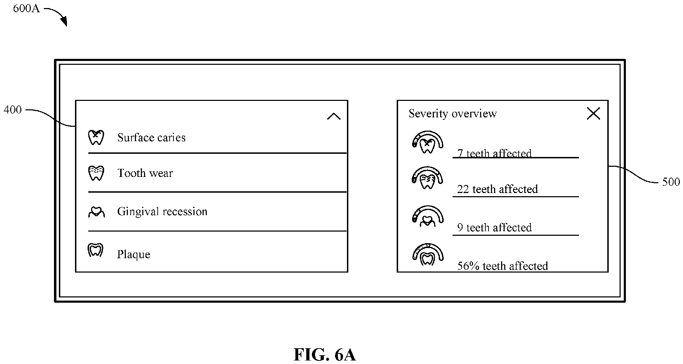

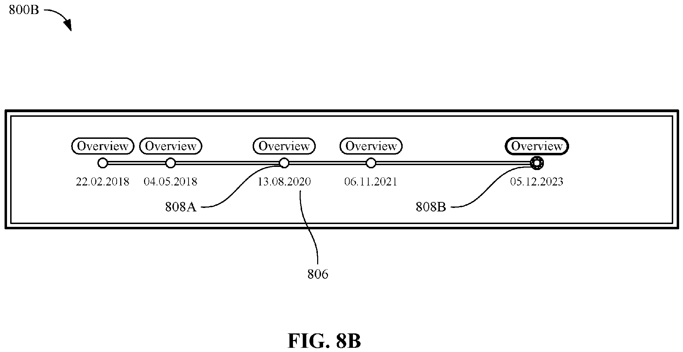

FIG. 6A shows a fifth exemplary variation of the first user interface 310A associated with the dentition 110, in accordance with an embodiment of the disclosure. Referring to FIG. 6A, a diagram 600A of the fifth exemplary variation of the first user interface 310A associated with the dentition 110 is shown. FIG. 6A may be explained in conjunction with elements from FIG. 1, FIG. 2, FIG. 3, FIG. 4A-FIG. 4E, and FIG. 5A-FIG. 5C. As shown in 600A, the first user interface 310A includes the one or more user-selectable interface elements 400 of the first user interface 310A and the severity overview information 500 associated with the first user interface 310A. In various embodiments of the disclosure, the first user interface 310A may include any combination of other variations of the one or more user-selectable interface elements 400 and/or the severity overview information 500. For example, the one or more user-selectable interface elements 400 and the severity overview information 500 may be separate elements in the first user interface 310A. The severity overview information 500 may be, in a preferred embodiment, displayed in association with a timeline of a second user interface 806 (FIG. 8B), more specifically in association with user-selectable timestamp interface elements 808A, 808B. In this way, by detecting user engagement of timestamp interface elements, the severity overview associated with a respective timestamp may be shown, allowing the efficient tracking of changes in severity distribution of various dental anomalies, thus serving as an importaint diagnostic aid. The user may engage the timestamp interface elements in various ways, including hovering over the elements and clicking on the elements. In another example, the severity overview information 500 may be integrated into the one or more user-selectable interface elements 400 (such as in FIG. 4B).

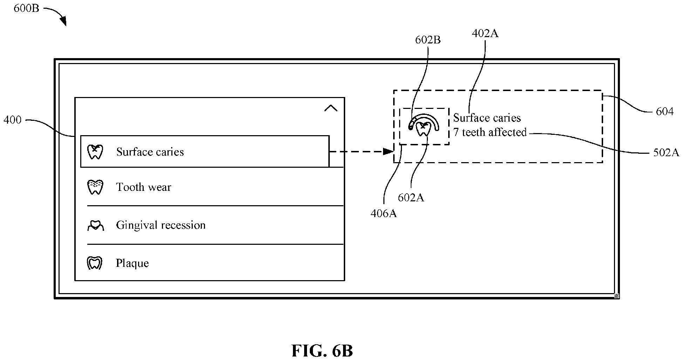

FIG. 6B shows a sixth exemplary variation of the first user interface 310A associated with the dentition 110, in accordance with an embodiment of the disclosure. Referring to FIG. 6B, a diagram 600B of the sixth exemplary variation of the first user interface 310A associated with the dentition 110 is shown. FIG. 6B may be explained in conjunction with elements from FIG. 1, FIG. 2, FIG. 3, FIG. 4A-FIG. 4E, FIG. 5A-FIG. 5C, and FIG. 6A. As shown in 600B, the first user interface 310A includes the one or more user-selectable interface elements 400 of the first user interface 310A. Further, in an embodiment, the severity overview information 500 is shown only for a selected dental anomaly through a corresponding user-selectable interface element, and not for all the dental anomalies among the plurality of dental anomalies. For example, a user selection of the dental anomaly from the user-selectable interface elements 400 is received via a specific interface element from the one or more user-selectable interface elements 400. The severity overview information 604 may be shown that is associated with the selected dental anomaly. For example, if the selection of the specific interface element corresponding to the surface caries anomaly is received from a user, then the severity overview information 604 may be shown within the first user interface 310A for giving additional severity related information associated with the surface caries anomaly. To this end, the severity overview information 604 may include textual information corresponding to the name of the dental anomaly, i.e., surface caries. In addition, the severity overview information 604 may include anomaly occurrence information 502A for the surface caries anomaly. Moreover, the severity overview information 604 may include a first visual representation 602A, such as a symbol corresponding to the surface caries anomaly, and a second visual representation 602B, such as a scale indicating the number of teeth affected and severity of the affected teeth. The first visual representation 602A (corresponding to 406B) and the second visual representation 602B (corresponding to 406C) are collectively represented within 406A.

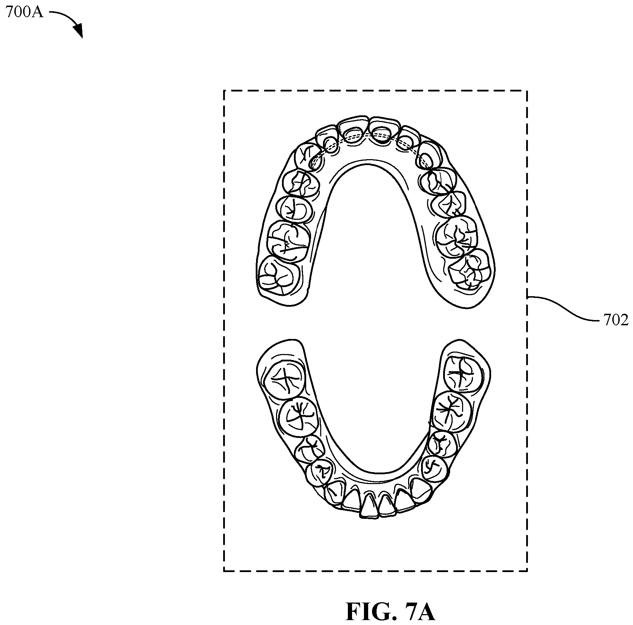

FIG. 7A shows a diagram 700A of a first exemplary variation of an anomaly view 702 associated with the first user interface 310A, in accordance with an embodiment of the disclosure. FIG. 7A may be explained in conjunction with elements from FIG. 1, FIG. 2, FIG. 3, FIG. 4A-FIG. 4E, FIG. 5A-FIG. 5C, FIG. 6A, and FIG. 6B. In an embodiment, the anomaly view 702 includes a 3D model of the dentition 110 with no indication of any of the plurality of dental anomalies when no dental anomaly is selected using the user-selectable interface elements 400. Alternatively, if dentition is healthy and the plurality of dental anomalies is a null set, then also the 3D model of the dentition 110 may be shown within the anomaly view 702 with no indication of any of the plurality of dental anomalies, indicating the dentition 110 as a healthy dentition. In an exemplary embodiment, in case a portion of the dentition 110 is not captured properly or the 3D model of a portion is not generated resulting in an inaccurate determination of dental anomaly for that portion, then that portion may be marked with a shape/colour/pattern that indicates 'insufficient quality' for determining the dental anomaly.

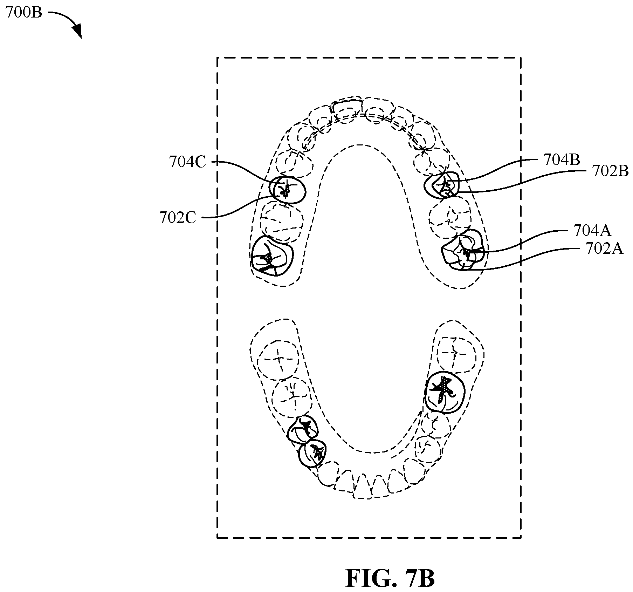

FIG. 7B shows a diagram 700B of a second exemplary variation of an anomaly view associated with the first user interface 310A, in accordance with an embodiment of the disclosure. FIG. 7B may be explained in conjunction with elements from FIG. 1, FIG. 2, FIG. 3, FIG. 4A-FIG. 4E, FIG. 5A-FIG. 5C, FIG. 6A- FIG. 6B, and FIG. 7A. After receiving the user selection of a first dental anomaly, say the surface caries anomaly among the plurality of dental anomalies through a first user-selectable interface element 402A or 404A from the one or more user-selectable interface elements 400, the system 102 may generate and show a first anomaly view of the dentition 110. In an example, generating the first anomaly view may include visually modifying at least a first portion of the 3D model within the 3D model. The first anomaly view may visually modify by highlighting certain portions of the dentition 110 in the 3D model indicating teeth that are affected by the selected dental anomaly. In an example, the highlighted portions in the first anomaly view may be shown in solid colours or patterns as compared to other portions of the 3D model of the dentition 110 in the anomaly view 702, where the other portions may be masked, shown in natural color or shown in translucent variation. In another example, the highlighted portions or teeth in the first anomaly view may be labelled as per one of the standard labelling conventions for the teeth. Further, in an embodiment, a highlighted tooth may include a portion of the tooth or complete area of the tooth highlighted in a particular colour/pattern indicating a severity category or an extent to which the portion of the tooth or the complete area of the tooth may be affected with the selected dental anomaly. For example, a tooth 702A may be affected with a selected dental anomaly surface caries. In an example, a tooth 702A that is severely affected by the surface caries anomaly may be shown in a colour or pattern 704A that indicates 'severe' category in an associated legend information, such as the legend information shown in 400E of FIG. 4E. Further, another tooth 702B that may be moderately affected by the surface caries anomaly may be shown in a colour or pattern 704B that indicates 'moderate' category. Accordingly, the associated legend information for the surface caries anomaly may also include the colour or pattern 704A marked again 'severe' category, and the colour/pattern 704B marked again 'moderate' category. Similarly, continuing further with the example, the tooth 702C that may be initially affected by the surface caries anomaly may be shown in a colour or pattern 704C that indicates 'initial' category. Thus, legend information for the surface caries anomaly may also include the colour/pattern 704C marked again 'initial' category.

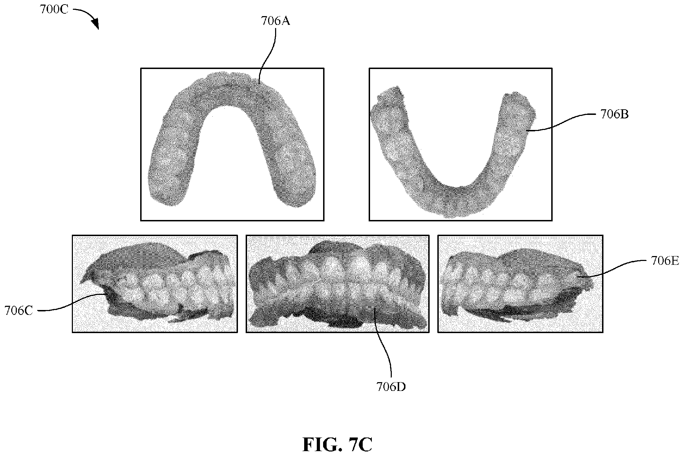

FIG. 7C shows a diagram 700C of a third exemplary variation of the anomaly view associated with the first user interface 310A, in accordance with an embodiment of the disclosure. FIG. 7C may be explained in conjunction with elements from FIG. 1, FIG. 2, FIG. 3, FIG. 4A-FIG. 4E, FIG. 5A-FIG. 5C, FIG. 6A-FIG. 6B, and FIG. 7A-FIG. 7B. In an embodiment, as shown in 700C, the anomaly view may include a set of one or more original images of the dentition 110. Accordingly, the anomaly view may include a first view 706A of an upper jaw of the dentition 110, a second view 706B of a lower jaw of the dentition 110, a left side view 706C of the dentition 110, a front view 706D of the dentition 110, and a right side view 706E of the dentition 110 overlaid on the first user interface 310A. In an example, when a specific interface element for a dental anomaly is selected, then teeth, tooth, or a portion of a tooth or multiple teeth may be highlighted within the first view 706A, the second view 706B, the left side view 706C, the front view 706D, and/or the right side view 706E to indicate tooth or teeth affected by the selected dental anomaly.

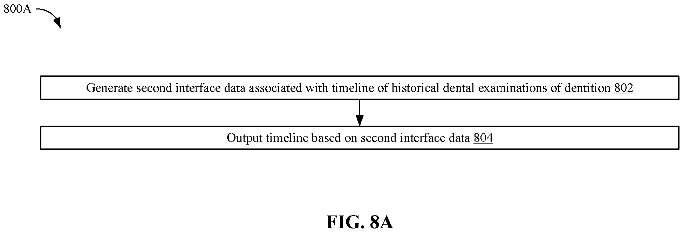

FIG. 8A is a flowchart 800A that illustrates exemplary operations for outputting a timeline, in accordance with an embodiment of the disclosure. FIG. 8A is explained in conjunction with elements from FIG. 1, FIG. 2, FIG. 3, FIG. 4A-FIG. 4E, FIG. 5A-FIG. 5C, FIG. 6A-FIG. 6B, and FIG. 7A-FIG. 7C. The exemplary operations illustrated in the flowchart 800 may start at 802 and may be performed by the system 102 of FIG. 1 or the processor 202 of FIG. 2. Although illustrated with discrete blocks, the exemplary operations associated with one or more blocks of the flowchart 800 may be divided into additional blocks, combined into fewer blocks, or eliminated, depending on the particular implementation.

At 802, the second interface data is generated. In an embodiment, the system 102 may be configured to generate the second interface data associated with a timeline of one or more historical dental examinations of the dentition 110. In an embodiment, the second interface data 308B may be generated using received scan data that may have been captured during one or more historical scanning sessions of the dentition 110. The second interface data 308B may include one or more user-selectable timestamp interface elements such as 804. Further, each of the one or more user-selectable timestamp interface elements may be selectable to output historical interface data associated with one of the one or more historical dental examinations of the dentition 110.

For example, the system 102 may receive a user input for selection of one of the user-selectable timestamp interface elements. For example, when the user input is received, the system 102 may output the first user interface associated with the dentition 110 of the time point that is selected using the user-selectable timestamp interface element. For example, the patient 112 visits the clinic of a dental practitioner for treatment of dentition 110, thrice on different dates, say, on 01-01-2020 (referred to as D1), 01-01-2021 (referred to as D2), and 01-01-2022 (referred to as the present date). So, the dates D1 and D2 are previous dates on which the patient visits the clinic. For example, the dates D1 and D2 are the dates of historical dental examinations of the dentition 110. Now, the second interface data may include the dates D1, D2, and the present date.

At 804, timeline is output based on the second interface data. In an embodiment, the system 102 may be configured to output the timeline based on the second interface data. In an embodiment, the output of the timeline includes display of the timeline via the display device 206A of the computer system 102.

In an example, the system 102 may be configured to control the display device 206A to display the timeline based on the second interface data. The timeline may be displayed with the first user interface 310A. Further, the user (or the dental practitioner in this example) may click or touch via the I/O interface 206 of the system 102 to access the historical data recorded on the dates of historical dental examinations of the dentition 110. For example, the user clicks on date D1, then the system 102 may fetch the historical interface data associated with a historical dental examination of the dentition 110 performed on the date D1. Further, the system 102 may output a second user interface corresponding to the timeline based on the second interface data. The second user interface may include, for example, the timeline as well as historical view elements corresponding to the historical dental examinations of the dentition 110 on the dates D1 and D2. In an example, the second user interface or the timeline may be overlaid on the first user interface 310A. In another example, the second user interface or the timeline may be displayed in a first part of a screen of the display device 206A and the first user interface 310A may be displayed in a second part of the screen of the display device 206A. In an example, after clicking the user-selectable timestamp interface element corresponding to a previous date, say D1, the first user interface 310A may be replaced with the historical view element corresponding to the date D1.

Accordingly, blocks of the flowchart 800 support combinations of means for performing the specified functions and combinations of operations for performing the specified functions. It will also be understood that one or more blocks of the flowchart 800 and can be implemented by special-purpose hardware-based computer systems which perform the specified functions, or combinations of special-purpose hardware and computer instructions.

Alternatively, the system 102 may include means for performing each of the operations described above. In this regard, according to an example embodiment, examples of means for performing operations may include, for example, the processor 202 and/or a device or circuit for executing the computer program instructions or executing an algorithm for processing information as described above.

Referring to FIG. 8B, a diagram 800B of an exemplary variation of a second user interface 806 associated with a timeline, in accordance with an embodiment of the disclosure. FIG. 8 may be explained in conjunction with elements from FIG. 1, FIG. 2, FIG. 3, FIG. 4A-FIG. 4E, FIG. 5A-FIG. 5C, FIG. 6A-FIG. 6B, FIG. 7A-FIG. 7C, and FIG. 8A. The second user interface 806 indicating the timeline is generated based on second interface data. The timeline may be associated with historical dental examinations of the dentition 110. The timeline may include one or more timestamps, where each timestamp indicates a time of a historical dental examination of the dentition 110. For example, the timestamps of the timeline are shown as user-selectable timestamp interface elements 808A and 808B. In various embodiments, the user-selectable timestamp interface elements 808A and 808B may be shown in other variations than circular dots, such as, triangular, square, rectangular, or images, icons, symbols, etc. In an embodiment, a user may click on a user-selectable timestamp interface element to access the data associated with the corresponding date of historical dental examination. In an embodiment, the information indicating the anomaly score of the plurality of dental anomalies, or a selected dental anomaly may be shown as the user hovers over the user-selectable timestamp interface elements 808A and 808B on the timeline. For example, the user may select a dental anomaly, say the surface caries anomaly from the user-selectable interface elements 400 in the first user interface 310A. In such a case, when the user hovers over a user-selectable timestamp interface element, say the timestamp interface element 808A, then the second user interface data may include a historical view data of a historical view of historical occurrence of the selected anomaly, i.e., the surface caries anomaly in the dentition 110. The historical view of the historical occurrence of the selected anomaly may be shown in conjunction with the first user interface 310A. Alternatively, when the user hovers over a user-selectable timestamp interface element, the corresponding anomaly data of the plurality of dental anomalies (irrespective of the selection of the dental anomaly) may be shown as a historical view in conjunction with the first user interface 310A. In an example, the timeline may include one or more timestamps indicating dates of historical examination and/or a present date when the current dental examination of the dentition 110 is carried out. For example, a date 804 may be a date of a historical dental examination. Moreover, each of the plurality of timestamps may be associated with a user-selectable timestamp interface element, for example, the user-selectable timestamp interface elements 808A and 808B. Further, when the user selects one of the user-selectable timestamp interface elements, the appearance of the user-selectable timestamp interface elements may change to indicate the selection, for example, the appearance of the user-selectable timestamp interface elements may change as shown in 808B which is different from 808A. In a prefered embodiment, the user-selectable timestamp interface elements may be associated with the severity overview (such as shown in FIG. 5A-5C) so that while the user hovers, e.g. with a mouse pointer, over one of the user-selectable timestamp interface elements, the severity overview corresponding to that timestamp is displayed. This functionality solves a technical problem of obtaining at a glance a specific severity overview of the dental anomalies, where the user does not need to separately select each anomaly individually from the user-selectable interface elements 400 in the first user interface 310A. In this way a visual aid for tracking progression of plurality of dental anomalies is provided. This design feature further contributes to maintaining user's focus while keeping the context of diagnostic findings intact.

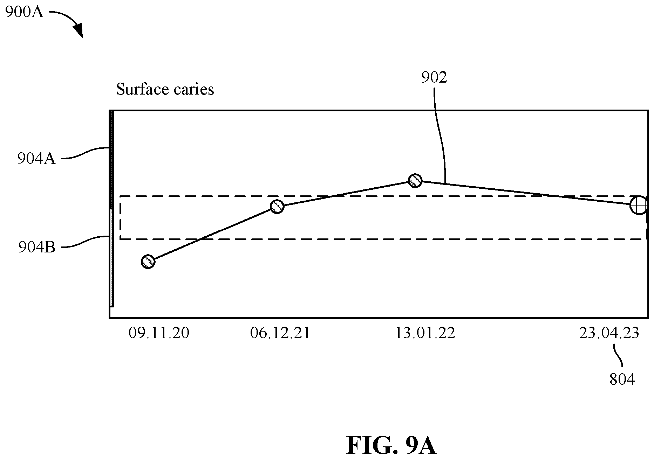

FIGs. 9A-9D show various exemplary elements of report data that may be included in a report associated with the second user interface, in accordance with various embodiments of the disclosure. FIGs. 9A-9D are explained in conjunction with elements from FIG. 1, FIG. 2, FIG. 3, FIG. 4A-FIG. 4E, FIG. 5A-FIG. 5C, FIG. 6A-FIG. 6B, FIG. 7A-FIG. 7C, and FIG. 8A-FIG. 8B. Pertinently, in various embodiments of the disclosure, the report data associated with the second user interface may be overlaid on the interface elements of the first user interface 310A that may be displayed via the display device 206A of the computer system 102.

Referring to FIG. 9A, a diagram 900A is shown. The diagram 900A shows a first exemplary element of the report data that may be included in the report. As shown in the diagram 900A, the report data may include trend information associated with a particular dental anomaly of the plurality of dental anomalies. The trend information shown in diagram 900A includes a graph 902 with nodes plotted for each of the dates of historical dental examination of the dentition 110, such as the date 804. Further, elements 904A and 904B may correspond to severity level of the dental anomaly with which the dentition 110 is affected. For example, the graph 902 corresponds to surface caries anomaly. In such case, horizontal graph area of 904A may correspond to 'severe' category of the teeth affected by the surface caries anomaly, and horizontal graph area of 904B may correspond to 'moderate' category of the teeth affected by the surface caries anomaly. Similarly, in an embodiment, the trend information for each of the plurality of dental anomalies associated with the dentition 110 may be included in the report data.

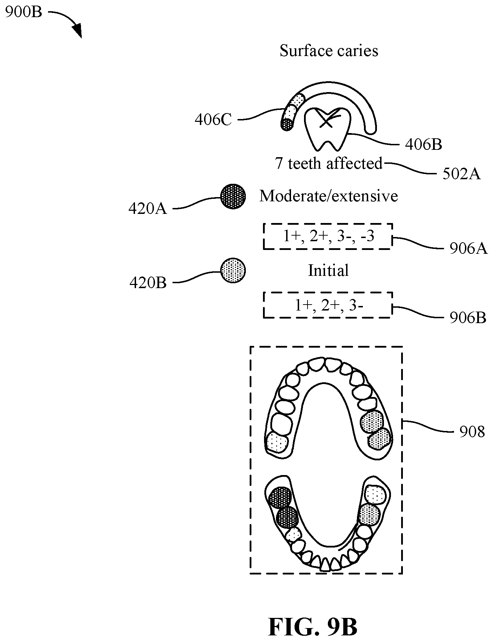

Referring to FIG. 9B, a diagram 900B is shown. The diagram 900B shows a second exemplary element of the report data that may be included in the report. As shown in diagram 900B, detailed information about tooth or teeth affected with a dental anomaly among the plurality of dental anomalies is shown. For example, the detailed information shown in 900B may correspond to the surface caries anomaly. As shown, the first visual representation 406B and the second visual representation 406C within the interface element 406A corresponding to the surface caries anomaly along with the severity overview information 502 may be provided. Further, the legend information including the severity categories along with the colour/pattern 420A and 420B indicating the corresponding severity category may be provided. Further, the actual teeth positions 906A and 906B of the affected teeth in the dentition 110 may also be provided for moderate/extensive and/or initial severe category respectively within the report data. Further, a representation 908 of the dentition 110 including the lower jaw of the dentition 110 and the upper jaw of the dentition 110 may be provided within the report data. The representation 908 of the dentition 110 may include the tooth or teeth marked with the corresponding colour/pattern indicating the severity category of the dental anomaly with which the teeth may be affected. Similarly, in an embodiment, corresponding detailed information for each of the plurality of dental anomalies associated with the dentition 110 may be included in the report data.

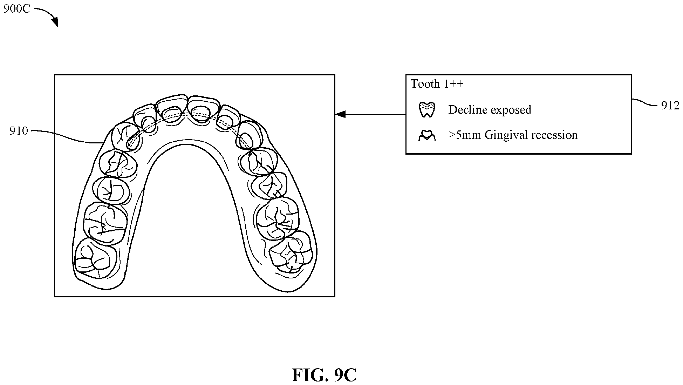

Referring to FIG. 9C, a diagram 900C is shown. The diagram 900C shows a third exemplary element of the report data that may be included in the report. In the diagram 900C, tooth information 912 of a tooth at position 1++ is shown. Notably, the tooth at the position 1++ is a part of the dentition 110 that is shown in 910. The tooth information 912 may include the information of the one or more dental anomalies among the plurality of dental anomalies with which the tooth at the position 1++ is affected. For example, the tooth at position 1++ is affected by the surface caries anomaly and the gingival recession anomaly, then the tooth information 912 may include the information indicating that the tooth at the position 1++ is affected by the surface caries anomaly and the gingival recession anomaly along with the severity extent for both the surface caries anomaly and the gingival recession anomaly. Similarly, the tooth information for each tooth of the dentition 110 or each affected tooth of may be included in the report data.

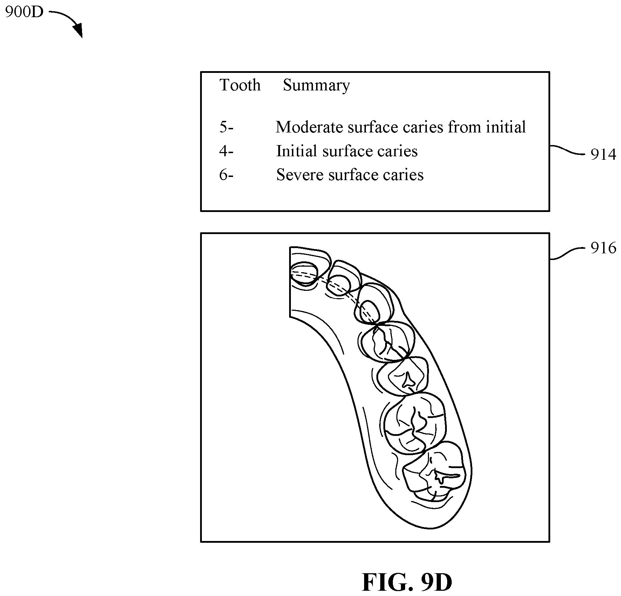

Referring to FIG. 9D, a diagram 900D is shown. The diagram 900D shows a fourth exemplary element of the report data that may be included in the report. In the diagram 900D, a diagnose summary 914 of one or more teeth in a portion 916 of the dentition 110 is shown. The diagnose summary 914 may be included in the report data of the report. As shown, the diagnose summary 914 may be provided in a tabular manner, for each of the affected teeth that are shown in 916. The diagnose summary 914 may include tooth position for each of the affected teeth that are shown in 916, in one column. Further, the diagnose summary 914 may include summary information against the corresponding tooth, where the summary information is associated with each of the plurality of dental anomalies for each of the affected teeth that are shown in 916. The summary information for a corresponding tooth may include one or more dental anomalies with which the corresponding tooth may be affected, and the severity extent for corresponding tooth for each of the one or more dental anomalies.

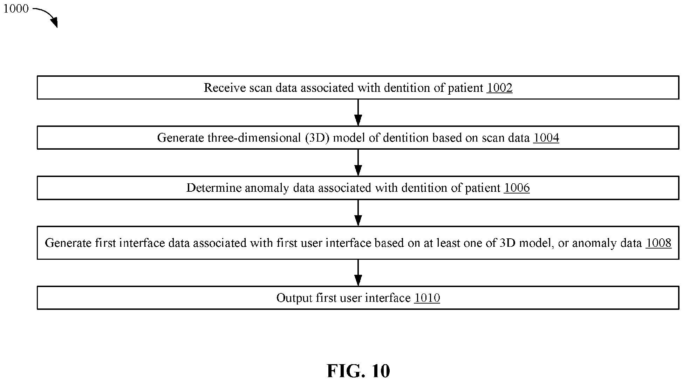

FIG. 10 is a flowchart 1000 that illustrates an exemplary method for outputting the first user interface 310A associated with the dentition 110, in accordance with an embodiment of the disclosure. FIG. 10 is explained in conjunction with elements from FIG. 1, FIG. 2, FIG. 3, FIG. 4A-FIG. 4E, FIG. 5A-FIG. 5C, FIG. 6A-FIG. 6B, FIG. 7A-FIG. 7C, FIG. 8A-FIG. 8B, and FIG. 9A-FIG. 9D. The exemplary operations illustrated in the flowchart 1000 may start at 1002 and may be performed by the system 102 of FIG. 1 or the processor 202 of FIG. 2. Although illustrated with discrete blocks, the exemplary operations associated with one or more blocks of the flowchart 1000 may be divided into additional blocks, combined into fewer blocks, or eliminated, depending on the particular implementation.

At 1002, the scan data 204A is received. In an embodiment, the system 102 may be configured to receive scan data 204A associated with the dentition 110 of the patient112. The scan data 204A may include the 2D images of the dentition 110 of the patient 112.

At 1004, a 3D model of the dentition 110 is generated based on the scan data 204A. In an embodiment, the system 102 may be configured to generate the 3D model of the dentition 110 based on the scan data 204A. At 1004, the system 102 may receive as an input, the scan data 204A. The system 102 may be configured to use the ML model(s) 204C for generating the 3D model based on the received scan data 204A or otherwise generate the 3D model. In an embodiment, the ML model 204C for generating the 3D model may be the first ML model. The first ML model may be configured to analyse the scan data 204A and generate a 3D model data 204B (also referred to as 3D model) of the dentition 110 using the scan data 204A associated with the dentition 110.

At 1006, anomaly data is determined. In an embodiment, the system 102 may be configured to determine anomaly data associated with the dentition 110 of the patient 112. The anomaly data is associated with at least one of a plurality of dental anomalies. In an example, the system 102 may be configured to determine the anomaly data associated with the dentition 110 based on the 3D model data 204B (or 3D model). In an embodiment, the system 102 may use the ML module 202C to determine the anomaly data associated with the dentition 110. The ML module 202C may execute a trained second ML model for determining the anomaly data for the dentition 110. The anomaly data may pertain to a plurality of dental anomalies. The plurality of dental anomalies may include, but is not limited to, a surface caries anomaly, an occlusal caries anomaly, a proximal caries anomaly, a tooth wear anomaly, a gingival recession anomaly, a gingivitis anomaly, and a plaque anomaly.

At 1008, the first interface data 308A is generated. In an embodiment, the system 102 may be configured to generate the first interface data 308A associated with the first user interface 310A based on the 3D model and/or the anomaly data. The first interface data 308A may be associated with the first user interface 310A. The first user interface 310A may include one or more user-selectable interface elements. Each of the one or more user-selectable interface elements may be selectable to generate an anomaly view of the dentition, such that the anomaly view indicates a corresponding dental anomaly of the plurality of dental anomalies within the 3D model.

At 1010, the first user interface 310A is output. In an embodiment, the system 102 may be configured to output the first user interface 310A based on the first interface data 308A. In an embodiment, the output includes display of the first user interface 310A via the display device 206A of the computer system 102.

Accordingly, blocks of the flowchart 1000 support combinations of means for performing the specified functions and combinations of operations for performing the specified functions. It will also be understood that one or more blocks of the flowchart 1000 and can be implemented by special-purpose hardware-based computer systems that perform the specified functions, or combinations of special-purpose hardware and computer instructions.

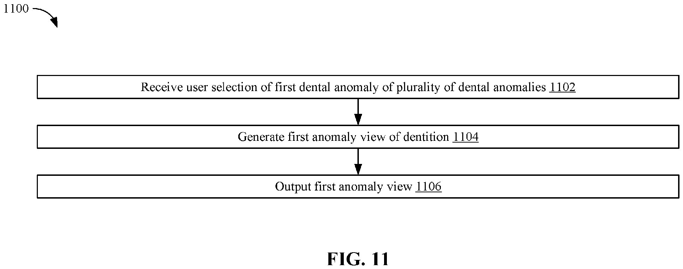

FIG. 11 is a flowchart 1100 that illustrates exemplary operations for outputting an anomaly view, in accordance with an embodiment of the disclosure. FIG. 11 is explained in conjunction with elements from FIG. 1, FIG. 2, FIG. 3, FIG. 4A-FIG. 4E, FIG. 5A-FIG. 5C, FIG. 6A-FIG. 6B, FIG. 7A-FIG. 7C, FIG. 8A-FIG. 8B, FIG. 9A-FIG. 9D, and FIG. 10. The exemplary operations illustrated in the flowchart 1100 may start at 1102 and may be performed by the system 102 of FIG. 1 or the processor 202 of FIG. 2. Although illustrated with discrete blocks, the exemplary operations associated with one or more blocks of the flowchart 1100 may be divided into additional blocks, combined into fewer blocks, or eliminated, depending on the particular implementation.

At 1102, a user selection is received. In an embodiment, the system 102 may be configured to receive the user selection of a first dental anomaly of the plurality of dental anomalies after displaying the first user interface 310A via the display device 206A. Since the one or more user-selectable interface elements correspond to the one or more dental anomalies that are determined in the dentition 110, the user selection of one of the user-selectable interface elements corresponds to the selection of a dental anomaly among the plurality of dental anomalies.

At 1104, a first anomaly view is generated. In an embodiment, the system 102 may be configured to generate the first anomaly view of the dentition 110. The first anomaly view may correspond to the anomaly view as explained with reference to FIGs. 7A-7C above in the disclosure. The first anomaly view may include at least a first portion of the 3D model, where the first portion of the 3D model indicates an occurrence of the first dental anomaly in the dentition 110 of the patient 112. Here, the first portion refers to one or more teeth or areas of the one or more teeth that are affected by the corresponding dental anomaly. In an embodiment, the first anomaly view may highlight the first portion in various ways including, but not limited to, adding a colour overlay layer on the 3D model, adding a pattern view layer on the 3D model, adding a contour, or a combination thereof.

At 1106, the first anomaly view is output. In an embodiment, the system 102 may be configured to output the first anomaly view. In an embodiment, the output of the first anomaly view includes display of the first anomaly view via the display device 206A of the computer system 102.

Accordingly, blocks of the flowchart 1100 support combinations of means for performing the specified functions and combinations of operations for performing the specified functions. It will also be understood that one or more blocks of the flowchart 1100 and can be implemented by special-purpose hardware-based computer systems that perform the specified functions, or combinations of special-purpose hardware and computer instructions.

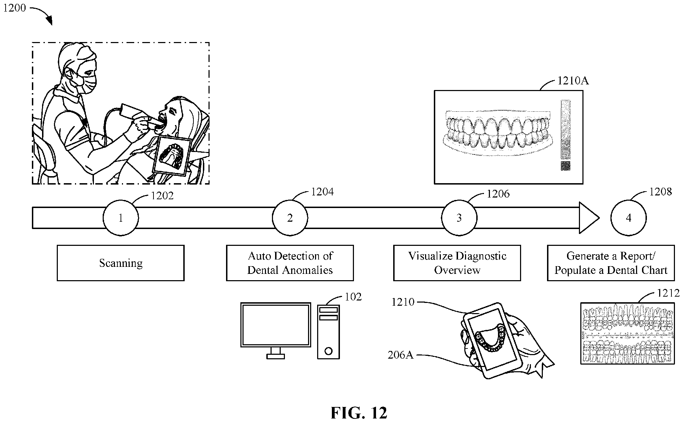

FIG. 12 is a diagram 1200 depicting overview of an exemplary method of generating a report, in accordance with various embodiments of the disclosure. As shown, at 1202, a scanning operation may be performed to obtain the scan data 204A. The scan data 204A may be obtained using a scanning device 106, such as, but not limited to, a handheld intraoral scanner. The computer system 102 is configured to receive the scan data 204A after the scan data 204A is obtained by the scanning device 106. Further details regarding scan data reception are provided at least with reference to FIG. 3. At 1204, the computer system 102 is configured to detect the dental anomalies in the dentition 110 using the received scan data 204A. Further details regarding anomaly data determination/detection of dental anomalies are provided at least with reference to FIG. 3. At 1206, the computer system 102 is configured to output a first user interface 1210A for visual diagnose overview of the dental anomalies. The first user interface 1210A may be an example embodiment of the first user interface 310A. The first user interface 1210A may be displayed via the display device 206A. The display device 206A may be integrated into a user device 1210 such as a smartphone, a computer, etc. Further details regarding the output of the user interface are provided at least with reference to FIG. 3. At 1208, the computer system 102 is configured to generate a report and/or populate a dental chart, such as, the chart 1212, for the user and/or the patient 112. In an embodiment, the computer system 102 may be configured to generate two different reports, one for the patient 112 and one for the user or a dentist. Further details regarding generating the report are provided at least with reference to FIG. 8A, FIG. 8B, and FIG. 9A- FIG. 9D.

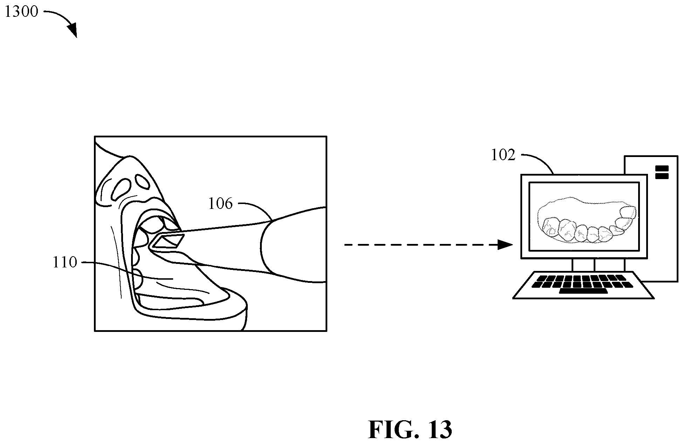

FIG. 13 is a diagram 1300 depicting overview of scanning of the dentition 110, in accordance with various embodiments of the disclosure. The scanning of the dentition 110 is performed using the scanning device 106. Further, the scan data 204A is transmitted to the computer system 102. The scanning device 106 may include a handheld intraoral scanner. The scanning device 106 may be configured to scan the dentition 110 of the patient 112. In an example, the handheld intraoral scanner may emit light of various wavelengths in a pulsating manner. For example, the images of the dentition 110 may be captured using light having different wavelengths, such as white light, blue light, IR light, NIR light, fluorescent light, etc. To this end, visible light images and/or IR images of the dentition 110 may be captured from a same position and at same time due to high pulse repetition rate of the light emitted from the handheld intraoral scanner. Also, the handheld intraoral scanner may include a projector unit configured to emit light at different wavelengths, such as at near-infrared wavelength, infrared wavelength, white-coloured wavelengths, and/or coloured visible wavelengths onto at least the dentition 110. The visible light emitted by the projector unit may include one or more colour lights of the filtered visible light signals, such as red, green, blue, and white. The projector unit may include multiple light sources that are configured to emit the one or more colours lights and the infrared light. The handheld intraoral scanner may further include an image sensor configured to capture the visible light information and the IR information from at least the dentition caused by the emitted light of the projector unit. The image sensor unit may include multiple cameras, such as, high speed cameras. The visible light information and IR information generated by the handheld intraoral scanner may be communicated to the system 102 over wired or wireless network 104.

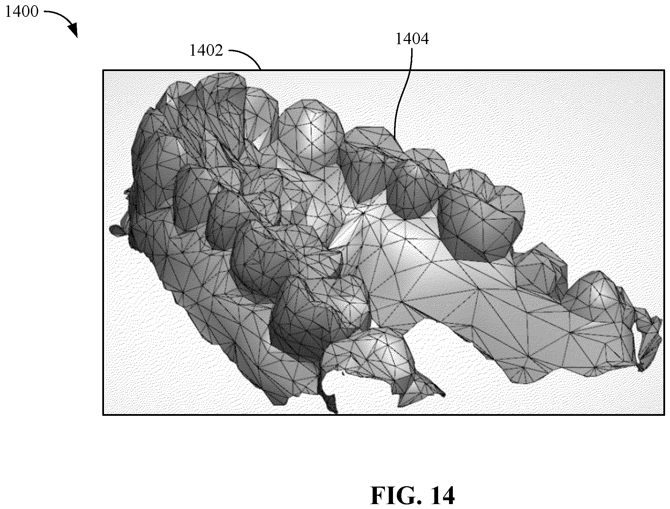

FIG. 14 is diagram 1400 depicting an exemplary 3D model 1402 of a portion of the dentition 110, in accordance with various embodiments of the disclosure. The 3D model 1402 may include a plurality of facets. A facet 1404 of the plurality of facets may refer to a flat, polygon surface element that makes up a mesh of the 3D model 1402. The plurality of facets of the 3D model 1402 may be represented by a point in a 3D point cloud of the 3D model 1402. Further, the 3D point cloud may include 3D points or 3D control points within voxels in a signed distance field, and the signed distance field may be converted into a 3D mesh for rendering the 3D model 1402. The 3D model 1402 may also include colour data and texture data of the surface of the teeth of the patient 112. Further, each facet of the plurality of facets may correspond to a quadrilateral forming a component of the mesh that approximates the surface of a scanned object, i.e., a tooth or teeth of the dentition 110. When the dentition 110 is scanned using the scanning device 106, the scan data 204A or a plurality of 2D images is processed to create an interconnected mesh of the plurality of facets. Such a mesh represents a surface geometry of the dentition 110 or a portion of the dentition 110 to create interconnected facets. Generally, a large number of facets, such as the facet 1404, may indicate a more detailed and accurate representation of the surface of the dentition 110 in the 3D model 1402.

FIG. 15A is a diagram 1500A that illustrates a first set of exemplary operations of a trained machine learning (ML) model for determining a dental anomaly, in accordance with an embodiment of the disclosure. The ML model may include a convolutional neural network (CNN). The CNN may include a plurality of convolution layers 1504 capable of capturing lowlevel features of an input 1502, i.e. obtaining convolved features. Moreover, one or more of pooling layers 1506 may be present. The one or more of pooling layers 1506 may be responsible for reducing the spatial size of convolved features. Further, a dense layer 1508 may apply a linear transformation to the input 1602, followed by a non-linear activation function, which enables the ML model to model intricate functions. CNN may be particularly suitable for processing matrix information such as 2D images. The input 1502 may be in a 3D format and may comprise at least one input element such as a tooth, a facet of a tooth, a point of a tooth point cloud, and/or a voxel. The input 1502 may be a 2D image of a tooth of the dentition 110 which has one of its surfaces affected by one of the plurality of dental anomalies, e.g. tooth wear. The input 1502 may alternatively comprise one or more 2D images of a surface of the tooth or at least one pixel of the 2D image of the tooth. An output 1510 of the CNN may include a probability value representing a likelihood that the dental anomaly, say the tooth wear anomaly is present in the input 1502 and/or may classify the dental anomaly further into a severity category using multi-class segmentation technique. For example, the tooth wear anomaly may be detected in the input 1502 and a moderate severity value may be assigned to it. Other exemplary severity categories may be "initial" or "severe".

FIG. 15B is a diagram 1500B that illustrates a second set of exemplary operations of a trained machine learning model for determining the dental anomaly, in accordance with an embodiment of the disclosure. The trained ML model may be a Point Net neural network capable of processing at least part of the 3D model in the form of a point cloud. A point cloud representation 1512A of a tooth of the 3D model may serve as an input 1512 to the trained ML model. The input 1512 may be of a format n x 3, where n is a number of 3D points representing the tooth and dimension '3' stands for three coordinates of each point representing the tooth. The input 1512 may be processed by an input transformation block 1514 which may be a transformation such as, but not limited to, a 3 x 3 rotation transformation. Further, a transformed input 1516 with a format n x 3 is generated. Alternatively, the input 1512 may be of a format n x 6, where n is the number of 3D points representing the tooth and dimension '6' stands for three coordinates of each point representing the tooth and three coordinates representing a normal of each point. In such a case, a first multi-layer perceptron 1518 with shared weights may process independently all 3D points of the 3D model of the dentition 110 for feature extraction. The first multi-layer perceptron (MLP) 1518 may be to achieve invariance under permutation of 3D points order. The first MLP 1518 may comprise an input layer of 3 dimensions, a hidden layer of 64 neurons and an output layer may comprise 64 features. Subsequently, an output 1524 of the first MLP 1518 may have a dimension of n x 64. Further, a feature transformation block 1522 may comprise a transformation matrix of dimensions 64 x 64, which may be a regularization matrix, for example, of L2 type. Subsequently, the output 1524 may be dimensioned as n x 64. The ML model may also include a second multi-layer perceptron (MLP) 1526 with shared weights. The second MLP 1526 may include an input layer of 64 features, two hidden layers of 64 neurons and 128 neurons, and an output layer of the second MLP 1526 may comprise 1024 features. Further, an output 1528 of the second MLP 1526 may have dimension of n x 1024. Further, the ML model may include a max pooling layer 1530. In the max pooling layer 1530, feature vectors of 3D points may be aggregated to create a 1024-dimensional global feature representation 1532 of the input 1512. The ML model may also include a third MLP 1534. The third MLP 1534 may comprise an input layer of 1024 features, two hidden layers of 512 neurons and 256 neurons, and an output layer of the third MLP 1534. An output of the third MLP 1534 may correspond to an output 1520 of the trained ML model. The output 1520 may include classification scores. The output 1520 of the trained ML model may comprise an indication that whether the dental anomaly, for example, the tooth wear anomaly, is present in the input 1512 or not, and/or may determine a location of the tooth wear in the input 1512. The output 1520 may further classify the tooth wear anomaly into a tooth wear type, for example one of abrasion, attrition, or erosion. In another example, the output 1520 may comprise additional information on the dental anomaly, including severity categories, for example, severe, moderate, or initial. In the case of tooth wear, additional information may include the number/identity of the affected tooth, a count of affected tooth surfaces, the tooth surface where the tooth wear lesion is located, etc. Further, a threshold value (p_th) for the probability value can be adjusted, based on which the dental anomaly may be detected using the trained ML model. In an example, behaviour of the trained ML model may be modified by adjusting a threshold value (p_th) for the probability value based on which determination of the dental anomaly may be made. By increasing the threshold value (closer to maximum value 100) the trained ML model may identify fewer dental condition lesions. By decreasing the threshold value (towards the minimum value 0) the trained ML model may identify more dental anomalies.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of reactants and/or functions, it should be appreciated that different combinations of reactants and/or functions may be provided by alternative embodiments without departing from the scope of the appended claims. In this regard, for example, different combinations of reactants and/or functions than those explicitly described above are also contemplated as may be set forth in some of the appended claims. Although specific terms are employed herein, they are used in a generic and descriptive sense only and not for purposes of limitation.

## Claims

1. A computer-implemented method (1000) for displaying an interface associated with a dentition (110), comprising:
receiving (1002) scan data (204A) associated with the dentition of a patient (112);
generating (1004) a three-dimensional (3D) model of the dentition based on the scan data;
determining (1006) anomaly data associated with the dentition of the patient based on the 3D model, wherein the anomaly data is associated with at least one dental anomaly of a plurality of dental anomalies;
generating (1008) first interface data (308A) associated with a first user interface (310A) based on the 3D model and/or the anomaly data, wherein the first user interface comprises one or more user-selectable interface elements, and wherein each interface element of the one or more user-selectable interface elements is selectable to generate an anomaly view (702) of the dentition, such that the anomaly view indicates a corresponding dental anomaly of the plurality of dental anomalies within the 3D model; and
outputting (1010) the first user interface based on the first interface data.

2. The computer-implemented method (1000) according to claim 1, further comprising:
receiving (1102) a user selection of a first dental anomaly of the plurality of dental anomalies, wherein the user selection is received based on the outputting of the first user interface (310A);
generating (1104) a first anomaly view of the dentition, wherein the first anomaly view comprises at least a first portion of the 3D model, and wherein at least the first portion of the 3D model indicates an occurrence of the first dental anomaly in the dentition (110) of the patient (112); and
outputting (1106) the first anomaly view.

3. The computer-implemented method (1000) according to any previous claim, further comprising causing to display, via a display device (206A), the first anomaly view, such that the first anomaly view is overlaid on the first user interface (310A).

4. The computer-implemented method (1000) according to any previous claim 2 or 3, wherein generating the first anomaly view comprises visually modifying at least the first portion of the 3D model within the 3D model.

5. The computer-implemented method (1000) according to any previous claim, wherein the anomaly data indicates an anomaly score associated with the at least one dental anomaly of the plurality of dental anomalies, the method further comprising:
receiving first visual representation data associated with the plurality of dental anomalies, the first visual representation data indicating a plurality of first visual representations, wherein each first visual representation of the plurality of first visual representations is associated with a dental anomaly of the plurality of dental anomalies;
generating second visual representation data associated with the plurality of dental anomalies based on the anomaly data, wherein the second visual representation data indicate a plurality of second visual representations of the anomaly score of the corresponding dental anomaly of the plurality of dental anomalies; and
generating the first interface data (308A) based on the first visual representation data and the second visual representation data.

6. The computer-implemented method (1000) according to claim 5, wherein generating the second visual representation data associated with a specific second visual representation of the plurality of second visual representations comprises:
determining an anomaly score associated with a specific dental anomaly of the plurality of dental anomalies based on the anomaly data associated with the specific dental anomaly;
generating categorisation data associated with the specific second visual representation for the specific dental anomaly based on the anomaly score, wherein the categorisation data indicates at least one of: one or more division areas of the specific second visual representation, a severity category from a plurality of severity categories for each of the one or more division areas, or a visual indicator from a plurality of visual indicators for each of the one or more division areas; and
generating the second visual representation data associated with the specific dental anomaly based on the categorisation data.

7. The computer-implemented method (1000) according to any previous claim 5 or 6, wherein each interface element of the one or more user-selectable interface elements indicates at least one of: a first visual representation of the plurality of first visual representations, or a second visual representation of the plurality of second visual representations, based on corresponding dental anomaly of the plurality of dental anomalies.

8. The computer-implemented method (1000) according to any previous claim 5-7, wherein each first visual representation of the plurality of first visual representations indicates a symbol associated with the corresponding dental anomaly of the plurality of dental anomalies, and wherein each second visual representation of the plurality of second visual representations indicates a scale for the anomaly score of the corresponding dental anomaly of the plurality of dental anomalies.

9. The computer-implemented method (1000) according to any previous claim, further comprising:
determining a timestamp associated with the first interface data (308A); and
storing the scan data (204A), the 3D model, the anomaly data and the first interface data in association with the timestamp.

10. The computer-implemented method (1000) according to claim 9, further comprising:
generating report data based on the anomaly data and the first interface data (308A), wherein the report data indicates at least one of: a health status of the dentition (110), or a treatment for the at least one dental anomaly of the plurality of dental anomalies; and
storing the report data in association with the timestamp.

11. The computer-implemented method (1000) according to any previous claim, further comprising:
generating (802) second interface data associated with a timeline of one or more historical dental examinations of the dentition, wherein the second interface data comprise one or more user-selectable timestamp interface elements (808A, 808B), and wherein timestamp interface element of the one or more user-selectable timestamp interface elements is selectable to output historical interface data associated with one of the one or more historical dental examinations of the dentition (110); and
outputting (804) the timeline based on the second interface data.

12. The computer-implemented method (1000) according to claim 11, further comprising:
causing to display, via a display device (206A), a second user interface (806) associated with the timeline based on the second interface data, wherein the second user interface is displayed with first user interface (310A).

13. A computer system (102) for displaying an interface associated with a dentition (110), the computer system comprising:
a memory (204) configured to store computer executable instructions; and
one or more processors (202) configured to execute the instructions to:
receive scan data (204A) associated with the dentition of a patient (112);
generate a three-dimensional (3D) model of the dentition based on the scan data;
determine anomaly data associated with the dentition of the patient based on the 3D model, wherein the anomaly data is associated with at least one dental anomaly of a plurality of dental anomalies;
generate first interface data (308A) associated with a first user interface (310A) based on the 3D modeland/or the anomaly data, wherein the first user interface comprises one or more user-selectable interface elements, and wherein each interface element of the one or more user-selectable interface elements is selectable to generate an anomaly view (702) of the dentition, such that the anomaly view indicates a corresponding dental anomaly of the plurality of dental anomalies within the 3D model; and
output the first user interface based on the first interface data.

14. The computer system (102) according to claim 13, further comprising a display device (206A), wherein the one or more processors (202) are further configured to execute the instructions to:
receive a user selection of a first dental anomaly of the plurality of dental anomalies, wherein the user selection is received based on the output of the first user interface (310A);
generate a first anomaly view of the dentition, wherein the first anomaly view comprises at least a first portion of the 3D model, and wherein at least the first portion of the 3D model indicates an occurrence of the first dental anomaly in the dentition of the patient; and
output the first anomaly view.

15. A computer program product for displaying an interface associated with a dentition (110), the computer program product comprising:
one or more computer-readable storage media; and
program instructions stored on the one or more computer-readable storage media to perform operations comprising:
receiving scan data (204A) associated with the dentition of a patient (112);
generating a three-dimensional (3D) model of the dentition based on the scan data;
determining anomaly data associated with the dentition of the patient based on the 3D model, wherein the anomaly data is associated with at least one dental anomaly of a plurality of dental anomalies;
generating first interface data (308A) associated with a first user interface (310A) based on the 3D model and/or the anomaly data, wherein the first user interface comprises one or more user-selectable interface elements, and wherein each interface element of the one or more user-selectable interface elements is selectable to generate an anomaly view (702) of the dentition, such that the anomaly view indicates a corresponding dental anomaly of the plurality of dental anomalies within the 3D model; and
outputting the first user interface based on the first interface data.
